# EUROPÄISCHE PATENTSCHRIFT

(11) **EP 3 116 590 B1**
(45) Veröffentlichungstag und Bekanntmachung des Hinweises auf die Patenterteilung: **01.01.2025**
(21) Anmeldenummer: 15709934.2
(22) Anmeldetag: 13.03.2015
(51) Int. Cl.: A61M 39/24

(54) **MEDIZINISCHE FUNKTIONSVORRICHTUNG MIT EINEM VENTILSITZ FÜR EIN REMANENTES RÜCKSCHLAGVENTIL**
MEDICAL DEVICE WICHT A VALVE SEAT FOR A REMANENT CHECK VALVE
DISPOSITIF MÉDICAL AVEC UN LOGEMENT POUR UNE SOUPAPE DE RETENUE RÉMANENTE

(30) Priorität: 14.03.2014 DE 102014103507
(43) Veröffentlichungstag der Anmeldung: 18.01.2017
(73) Patentinhaber: Fresenius Medical Care Deutschland GmbH, 61352 Bad Homburg (DE)
(72) Erfinder: LAUER, Martin, 66606 St. Wendel (DE)
(74) Vertreter: Bobbert & Partner Patentanwälte PartmbB
(86) Internationale Anmeldenummer: PCT/EP2015/055298
(87) Internationale Veröffentlichungsnummer: WO 2015/136076

(56) Entgegenhaltungen:
- WO-A1-2010/102784
- DE-A1- 102009 024 469
- DE-A1- 102010 032 181
- JP-A- S63 135 670
- US-A1- 2004 267 228
- US-A1- 2010 274 169

## Beschreibung

Die vorliegende Erfindung betrifft eine medizinische Funktionsvorrichtung mit den Merkmalen des Anspruchs 1.

Einmalteilsysteme werden in der Medizin- oder Labortechnik zunehmend als kompakte medizinische Funktionsvorrichtungen wie Kassettensysteme oder Blutkassetten ausgeführt, in denen Flüssigkeiten und Gase, insbesondere medizinische Flüssigkeiten und Blut, in Kanälen und Kammern geführt werden.

Das Funktionselement des Rückschlagventils kommt in solchen Kassettensystemen, aber auch als Einzelanordnung eingefügt in Schläuche, gerne zum Einsatz, um den Durchfluss von Fluiden passiv, also ohne Vorhandensein aktiv angetriebener Ventile, in der gewünschten Gegendurchflussrichtung zu sperren und in der gewünschten Durchflussrichtung freizugeben, häufig erst ab einem bestimmten Öffnungsdruck.

Manche Kassettendisposable (auch als Einwegkassetten bezeichnet) der Anmelderin der vorliegenden Anmeldung bestehen im Wesentlichen aus einem mittels Spritztechnik hergestellten Hartteil mit fluidführenden Strukturen und einer Folie. Die Folie verschließt nach ihrer Verpressung, Verklebung oder Verschweißung mit dem Hartteil halbseitig offene Hartteilkanäle und -kammern flüssigkeitsdicht. Die Folie ermöglicht neben der Verschlussfunktion aufgrund ihrer Nachgiebigkeit und dünnen Wandstärke auch das Messen von Parametern wie Temperaturen, Drücken und Füllständen, das Detektieren von Luft und Blut sowie das Betätigen von Ventilen und das Aktivieren von Rückschlagventilen durch die Folie hindurch. Solche Rückschlagventile können in solchen Kassetten dazu dienen, den Rückfluss von kontaminierter Dialyseflüssigkeit in den Hydraulik- oder Wasserabschnitt der Behandlungsmaschine (kurz: Maschine) zu verhindern, den Austritt von Blut über den arteriellen Heparin-Luer-Anschluss und den venösen Zugabe-Luer-Anschluss bei Fehlfunktionen der Maschine oder Fehlbedienungen durch den Anwender zu verhindern, das Eindringen von Luft in die Kassette unterhalb eines gegebenen Öffnungsdrucks (Unterdruck zur Umgebung) zu vermeiden und das Ausfließen von kontaminierten Restflüssigkeiten beim Abrüsten des Disposable in die Umgebung zu verhindern.

Die meisten Disposablesysteme müssen sterilisiert werden, wobei je nach Disposable-Werkstoffen neben den Strahlenbehandlungen Gamma und Ebeam auch Gasbehandlungen wie die ETO-, die Dampf-Vakuum- und die H₂O₂-Sterilisation zum Einsatz kommen. Bei den Gassterilisationen besteht das Problem, dass sich flächig berührende Oberflächen und versperrte oder gedrosselte Strömungswege die Sterilisation erschweren oder verunmöglichen können. Manche Rückschlagventile besitzen zur Gewährleistung der Gassterilisierbarkeit daher eine erste, anfängliche, durchflussoffene "Initial"-Stellung. Aus der Initialstellung wird das Rückschlagventil beim Aufrüsten der Behandlungsvorrichtung mittels der Kassette in eine zweite Stellung, die sog. "Aktiviert"-Stellung übergeführt. Erst ab diesem Überführen, hier als "Aktivierung" bezeichnet, weist das Ventil die Rückschlagfunktion auf, nicht aber bereits zuvor. Ein solches aktivierbares Rückschlagventil geht beispielsweise aus der Offenlegungsschrift DE 10 2009 024 469.7, eingereicht am 10. Juni 2009 beim Deutschen Patent- und Markenamt für die Anmelderin der vorliegenden Anmeldung, hervor.

Das o. g. aktivierbare Rückschlagventil wird nach Einbau der Blutkassette in die Dialysemaschine beim Schließen der Maschinentür aktiviert. Die Aktivierung geschieht dadurch, dass das Rückschlagventil mittels eines aus der Aktor-Sensor-Einheit hervorstehenden Höcker unter Einbeulung der Disposablefolie so weit in den Ventilsitz der Kassette verschoben wird, dass die ringförmigen Dichtsitzbereiche der Elastomerkomponente und der Hartkomponente mit Vorspannung aufeinander zu liegen kommen. Hierdurch wird die fluidoffene Initialstellung (hierin auch als erste Stellung bezeichnet) verlassen und die Rückschlagventilstellung (hierin auch als zweite Stellung bezeichnet) eingenommen. Die Klemm-ZentrierRippen der Elastomerkomponente weisen ein nur geringes Übermaß zur zugehörigen Klemm-Zentrier-Bohrung im Hartteil auf. So wird zunächst die sichere Gas-Sterilisation gewährleistet. Anschließend wird die zuverlässige Rückschlagfunktion aktiviert.

Die erfindungsgemäße Aufgabe besteht darin, eine weitere medizinische Funktionsvorrichtung mit wenigstens einem Rückschlagventil (hierin auch als Rückschlagventilanordnung bezeichnet) vorzuschlagen.

Die Aufgabe kann gelöst werden durch eine medizinische Funktionsvorrichtung mit den Merkmalen des Anspruchs 1.

Erfindungsgemäß wird somit eine medizinische Funktionsvorrichtung mit einem Ventilsitz für ein Rückschlagventil, auch als Rückschlagventilanordnung oder kurz als Ventil bezeichnet, vorgeschlagen.

Das Rückschlagventil ist ausgestaltet, um neben einer ersten, zum Gassterilisieren geeigneten Stellung, bei welcher ein Sterilisierungsspalt verbleibt, eine zweite Funktionsstellung einzunehmen. Dies geschieht mittels Aufbringens einer Kraft auf einen Abschnitt des Rückschlagventils und/oder eines Verschiebens des Abschnitts. Die erste Funktionsstellung wird hierin auch initiale Stellung oder Sterilisierungsstellung genannt. Die zweite Funktionsstellung wird hierin auch aktivierte Stellung oder Rückschlagstellung genannt.

In der zweiten Funktionsstellung übt das Rückschlagventil eine Rückschlagfunktion aus, wobei das Rückschlagventil ausgestaltet ist, um nach Wegfall der Kraft nach erfolgtem Überführen in die zweite Stellung hinein in der zweiten Stellung zu verbleiben.

Nach Wegfall der überführenden Kraft oder nach Lösen des verschiebenden Aktors vom Rückschlagventil zeigt das Rückschlagventil somit keine, jedenfalls aber keine ausreichende Rückstellwirkung, mittels welcher das Rückschlagventil von sich aus aus der Rückschlagstellung zurück in die Sterilisierungsstellung verbracht werden würde. Das Rückschlagventil kann als selbsthaltend bezeichnet werden.

Der Übergang von der ersten Stellung in die zweite Stellung wird auch hierin als "Aktivierung" bezeichnet.

Ein Rückschlagventil, welches selbst nach Wegfall jener Kraft, welche den Übergang von erster in zweite Stellung bewirkt hat, in der zweiten Stellung verbleibt, wird hierin auch als "remanent aktiviert" bezeichnet. Ein hierzu geeignetes Ventil wird hierin folglich auch als "remanent aktivierbar" angesehen und/oder bezeichnet.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale in beliebiger Kombination aufweisen.

In allen erfindungsgemäßen Ausführungsformen kann die Kappe als Rastkappe ausgestaltet sein.

In manchen erfindungsgemäßen, beispielhaften Ausführungsformen der Funktionsvorrichtung erfolgt die Betätigung des Ventils generell durch Aufprägen eines Wegs/einer Verschiebung. Die hierin genannte Kraft ist dann eine Reaktion hierauf.

In einigen erfindungsgemäßen, beispielhaften Ausführungsformen der Funktionsvorrichtung hat das Rückschlagventil in der ersten Stellung keine Rückschlagfunktion.

In manchen erfindungsgemäßen, beispielhaften Ausführungsformen der Funktionsvorrichtung ist das Rückschlagventil ausgestaltet, um mittels eines Aktors einer Blutbehandlungsvorrichtung von der ersten Stellung in die zweite Stellung übergeführt zu werden. Dabei wird die Funktionsvorrichtung zu deren Betrieb mit der Blutbehandlungsvorrichtung verbunden.

In einigen erfindungsgemäßen, beispielhaften Ausführungsformen ist die Funktionsvorrichtung derart ausgestaltet, dass das Rückschlagventil nach Wegfall der Kraft durch Reibschluss in der zweiten Stellung verbleibt. Hierzu kann ein Element vorgesehen sein, welches einen Reibschluss beispielsweise zwischen wenigstens einem Abschnitt eines Ventilkörpers und wenigstens einem Abschnitt eines Ventilsitzes gewährt. Dieses Element kann als Reibschlusselement bezeichnet werden.

In manchen erfindungsgemäßen, beispielhaften Ausführungsformen der Funktionsvorrichtung weist das Rückschlagventil einen Ventilkörper und/oder eine Kappe, welche, wann immer hierin erwähnt, optional als Rastkappe ausgestaltet sein kann, auf oder besteht hieraus; in diesen Ausführungsformen zählt der Ventilsitz nicht zum Rückschlagventil. In anderen erfindungsgemäßen, beispielhaften Ausführungsformen zählt hingegen auch der Ventilsitz zum Rückschlagventil.

In bestimmten erfindungsgemäßen, beispielhaften Ausführungsformen ist die Funktionsvorrichtung derart ausgestaltet, dass das Rückschlagventil nach Wegfall der Kraft nur durch Reibschluss in der zweiten Stellung verbleibt.

In gewissen erfindungsgemäßen, beispielhaften Ausführungsformen ist die Funktionsvorrichtung derart ausgestaltet, dass das Rückschlagventil (oder der Ventilkörper und/oder die Kappe) beim oder zum Überführen des Rückschlagventils aus der ersten Stellung in die zweite Stellung relativ zum Ventilsitz oder zur übrigen Funktionsvorrichtung bewegt wird. Die Bewegung relativ zum Ventilsitz kann dabei in einer Richtung erfolgen, welche vorzugsweise vollständig oder im Wesentlichen senkrecht zur Folienebene steht.

In bestimmten erfindungsgemäßen, beispielhaften Ausführungsformen ist die Funktionsvorrichtung derart ausgestaltet, dass die Kappe, soweit vorhanden, sich beim Öffnen des Rückschlagventils nicht relativ zum Ventilsitz bewegt.

In manchen erfindungsgemäßen, beispielhaften Ausführungsformen der Funktionsvorrichtung weist das Rückschlagventil einen Ventilkörper und eine hiervon getrennt gefertigte Kappe oder Rastkappe auf.

In einigen erfindungsgemäßen, beispielhaften Ausführungsformen der Funktionsvorrichtung weist der Ventilkörper eine Anzahl von Noppen auf. Diese ragen nach Verbinden des Ventilkörpers mit der Kappe radial aus Durchbrüchen der Kappe nach außen.

In bestimmten erfindungsgemäßen, beispielhaften Ausführungsformen der Funktionsvorrichtung weisen sowohl der Ventilkörper als auch die Kappe Drainagestrukturen auf.

In manchen erfindungsgemäßen, beispielhaften Ausführungsformen der Funktionsvorrichtung ist innerhalb des zylinderförmigen Abschnitts des Ventilsitzes der Kassette im Halteringbereich der Kassette eine Stufe oder eine stufenartige Durchmesserverringerung ausgestaltet.

In einigen erfindungsgemäßen, beispielhaften Ausführungsformen der Funktionsvorrichtung sind der Ventilsitz und/oder der Dichtabschnitt im Dichtungsbereich konisch oder flach ausgestaltet. Gegen diesen dichten der Ventilkörper und/oder die Kappe in der zweiten Stellung.

In bestimmten erfindungsgemäßen, beispielhaften Ausführungsformen der Funktionsvorrichtung sitzt der Ventilkörper radial unter einer ersten Vorspannung und axial unter einer zweiten Vorspannung in der Kappe. Dabei kann die zweite Vorspannung größer als die erste sein.

In manchen erfindungsgemäßen, beispielhaften Ausführungsformen der Funktionsvorrichtung weist der Ventilkörper die Form eines Napfes mit Ventilboden und optional einen zentral und beispielsweise steif an den Ventilboden angebundenen Zuganker auf. Dabei ist der Zuganker zum Verbinden des Ventilkörpers und der Kappe ausgestaltet, indem der Zuganker in eine Rastöffnung der Kappe oder Rastkappe eingerastet wird.

In einigen erfindungsgemäßen, beispielhaften Ausführungsformen der Funktionsvorrichtung weist die Kappe Zungen auf, welche sich radial erstrecken.

In bestimmten erfindungsgemäßen, beispielhaften Ausführungsformen der Funktionsvorrichtung sind der Zuganker und ein Ventilboden ausreichend steif ausgestaltet, so dass der Zuganker in der Kappe in der zweiten Stellung in alle Raumrichtungen berührungsfrei Abstand zu weiteren Abschnitten hält.

In manchen erfindungsgemäßen, beispielhaften Ausführungsformen der Funktionsvorrichtung sind der Zuganker und der Ventilboden ausreichend steif ausgestaltet, so dass der Zuganker in der zweiten Stellung in alle Raumrichtungen berührungsfrei Abstand in der Kappe zu allen Abschnitten hält.

In einigen erfindungsgemäßen, beispielhaften Ausführungsformen der Funktionsvorrichtung weist die Kappe die Form eines Gewölbes mit mehreren radial außen und axial oben offenen Durchbrüchen auf. Ferner sind in den Durchbrüchen Rastzungen angeordnet, die, wenn sie radial nach innen gebogen sind, die Durchbrüche nur teilweise verschließen.

In bestimmten erfindungsgemäßen, beispielhaften Ausführungsformen der Funktionsvorrichtung ist die Anzahl der Durchbrüche und der Rastzungen jeweils ungerade.

In einigen erfindungsgemäßen, beispielhaften Ausführungsformen der Funktionsvorrichtung ist das Rückschlagventil ausgestaltet, um nach erfolgtem Überführen in die zweite Stellung hinein einerseits und nach Wegfall der Kraft oder nach Lösen des verschiebenden Aktors vom Rückschlagventil andererseits aufgrund eines Verrastens, Verklemmens, Verkeilens oder dergleichen von Komponenten des Rückschlagventils miteinander in der zweiten Stellung zu verbleiben. Bei den Komponenten kann es sich um den Ventilkörper und die Kappe handeln, wobei sich der Ventilkörper durch Aufbringen der Kraft oder mittels des verschiebenden Aktors elastisch verformt in der Kappe verrastet, verklemmt, verkeilt, oder dergleichen. In bestimmten erfindungsgemäßen Ausführungsformen ist es dabei ein Haltering des Ventilkörpers, welcher sich hinter oder unter Abschnitten der Kappe, wie etwa den optionalen Auflagezungen der Kappe, verrastet, verkeilt, verklemmt oder dergleichen, während ein weiterer Abschnitt des Ventilkörpers, etwa ein optionaler Zentrierkonus hiervon, unter Wirkung eines Federelements gegen einen wiederum weiteren Abschnitt der Kappe, etwa eine optionale Konus-Aufnahme, drückt.

Das Federelement ist in bestimmten erfindungsgemäßen, beispielhaften Ausführungsformen der Funktionsvorrichtung als ein elastischer Abschnitt, vorzugsweise des Ventilkörpers, ausgestaltet. Das Federelement kann eine Federmembran sein. Die Federmembran kann umlaufend und/oder rotationssymmetrisch sein. Die Federmembran kann in einem Schnitt einen becherförmigen Abschnitt haben. Die Federmembran kann in einem Schnitt konzentrische Abschnitte aufweisen.

In einigen erfindungsgemäßen, beispielhaften Ausführungsformen der Funktionsvorrichtung ist das Rückschlagventil oder ein Abschnitt hiervon ausgestaltet, um eine Vorspannung des in den Ventilsitz eingefügten Rückschlagventils oder wenigstens eines Abschnitts hiervon zu bewirken.

In manchen erfindungsgemäßen, beispielhaften Ausführungsformen der Funktionsvorrichtung wird die Vorspannung mittels eines Federelements bewirkt, welche beispielsweise als Federmembran ausgestaltet ist.

In einigen erfindungsgemäßen, beispielhaften Ausführungsformen der Funktionsvorrichtung ist das Federelement ein Abschnitt des Ventilkörpers.

In manchen erfindungsgemäßen, beispielhaften Ausführungsformen der Funktionsvorrichtung weist die Kappe eine umlaufende Kante auf. Diese befindet sich auf Höhe der Rastzungen und bildet dabei die Haupttrennebene des Spritzgusswerkzeugs. Die umlaufende Kante kann in bestimmten erfindungsgemäßen Ausführungsformen eine Abweichung der Kappe von der Zylinderform bewirken, was vorteilhaft ein Verkanten oder Verklemmen der Kappe verhindern kann, wenn die Kappe gekippt oder Kippbewegungen ausgesetzt wird.

In einigen erfindungsgemäßen, beispielhaften Ausführungsformen der Funktionsvorrichtung verbleibt in der ersten Stellung ein umlaufender Spalt, hier als Sterilisations-Spalt bezeichnet. Diese axiale Einbaulage und damit der Sterilisations-Spalt werden durch Reibschluss von einander berührenden Komponenten, vorzugsweise von Ventilsitz einerseits und Ventilkörper andererseits, beispielsweise in Gestalt von Klemm-ZentrierRippen als Beispiel eines Reibschlusselements, aufrecht gehalten. Der Sterilisations-Spalt kann in der ersten Stellung von Sterilisationsfluid durchströmt werden. Er ist in der zweiten Stellung vorzugsweise geschlossen oder nicht mehr existent.

In bestimmten erfindungsgemäßen, beispielhaften Ausführungsformen ist die Funktionsvorrichtung als Blutkassette, Kassette, Blutschlauch oder Infusionsschlauch ausgestaltet.

In manchen erfindungsgemäßen, beispielhaften Ausführungsformen ist die Funktionsvorrichtung als Blutkassette ausgestaltet, welche einen Hartkörper und eine den Hartkörper oder Teile hiervon abdeckende Folie aufweist. Dabei ist der Ventilsitz im Hartkörper vorgesehen. Das Rückschlagventil ist angeordnet, um mittels Druck oder Verschiebung eines Aktors der Blutbehandlungsvorrichtung auf die Folie in die zweite Stellung übergeführt zu werden.

In einigen erfindungsgemäßen, beispielhaften Ausführungsformen der Funktionsvorrichtung ragt die folienseitige Stirnfläche des Rückschlagventils nicht über die Folienebene der Blutkassette hinaus.

In bestimmten erfindungsgemäßen Ausführungsformen ist ein einteiliger Ventilkörper aus Silikonkautschuk gefertigt. Der Ventilköper kann einen, vorzugsweise kelchförmigen, Radialdichtsteg aufweisen, welcher umlaufend fluiddicht an einen Kernzylinder angebunden ist.

In bestimmten erfindungsgemäßen Ausführungsformen weist der Ventilkörper Führungs-Klemm-Drainage-Rippen auf, welche zum Führen, zum Klemmen und zum Drainieren dienen. Diese sind vorzugsweise in gleichmäßiger und ungeradzahliger Teilung und in bevorzugter Anzahl von mehr als 2 an den Kernzylinder angebunden.

Der Ventilsitz kann in bestimmten erfindungsgemäßen Ausführungsformen zwei aneinander anschließende Zylinder, hier als Führungszylinder (unten im Ventilsitz) und Dichtzylinder (oben um Ventilsitz) aufweisen, wobei der Dichtzylinder einen größeren Durchmesser als der Führungszylinder hat, und wobei der Bereich des Durchmesserübergangs oder -sprungs zwischen beiden Zylindern als Raststufe bezeichnet wird. Zumindest einige der Klemm-Führungs-Noppen des Ventilkörpers oder der Führungs-Klemm-Drainage-Rippen liegen in der ersten Stellung am Führungszylinder an, die Klemm-Rast-Noppen, welche radial über den vorgenannten Klemm-Führungs-Noppen hervorragen, liegen in der ersten Stellung am Dichtzylinder an. Sowohl Klemm-Rast-Noppen als auch vorgenannten Klemm-Führungs-Noppen liegen in der zweiten Stellung am Führungszylinder an.

In bestimmten erfindungsgemäßen Ausführungsformen steht ein Radialdichtsteg des Ventilkörpers axial außer Eingriff zum Dichtzylinder, weshalb ein strömungsoffenes Ringvolumen (ein Sterilisierungsspalt) besteht. Erst durch Einleitung einer axialen Mindestaktivierungskraft (bevorzugt 20 bis 40 N) auf die Stirnfläche des Kernzylinders setzt eine axiale Verschiebebewegung ein.

Drainage-Fußrippen sitzen in bestimmten erfindungsgemäßen Ausführungsformen in der zweiten Stellung auf einem unteren Abschnitt, beispielsweise dem Boden, des Führungszylinders auf. Sie können hierdurch den möglichen Aktivierungshub begrenzen und zusammen mit allen anderen Rippen die gleichmäßige Entlüftbarkeit und Umströmbarkeit des Ventilkörpers sowie die sichere Sterilisierbarkeit mittels Gasen gewährleisten.

In bestimmten erfindungsgemäßen Ausführungsformen bildet ein umlaufender Radialdichtsteg mit vorzugsweise spitz zulaufender Dichtkante zusammen mit dem Dichtzylinder des Ventilsitzes der Kassette ein Rückschlag-Ventil-Dicht-System, nachdem der Aktivierungshub ausgeführt wurde. Dabei liegt die umlaufende Dichtkante des - vorzugsweise elastomeren - Ventilkörpers unter radialer Vorspannung am Dichtzylinder an.

In bestimmten erfindungsgemäßen Ausführungsformen besteht der Ventilkörper aus einem elastomeren Werkstoff, vorzugsweise aus Silikonkautschuk.

In bestimmten erfindungsgemäßen Ausführungsformen weist ein Positionierungsring des Ventilkörpers mehrere, vorzugsweise umlaufend angeordnete Drainagestrukturen auf, welche im eingebauten Zustand die Zugänglichkeit für Sterilisiergase gewährleisten.

In bestimmten erfindungsgemäßen Ausführungsformen ist eine obere Ringstirnfläche des Positionierungsrings bündig mit der Folie bzw. dem Kassettenrand, auf den die Folie aufgelegt oder aufgeschweißt ist.

In bestimmten erfindungsgemäßen Ausführungsformen weist eine äußere Mantelfläche des Positionierungsrings einen größeren Durchmesser auf als die zugeordnete Absatzbohrung in der Kassette. Über den Durchmesserunterschied, die Materialhärte und/oder die Gestaltung der Drainagestrukturen kann sich die reibungsverursachte Haltekraft in der Kassette ergeben.

In bestimmten erfindungsgemäßen Ausführungsformen dient eine untere Ringstirnfläche des Positionierungsrings als Bewegungsanschlag und damit der Kalibrierung des Öffnungsdrucks und der Durchlasskennlinie in der zweiten Stellung ("aktiviert").

In bestimmten erfindungsgemäßen Ausführungsformen besteht eine Durchlassringzone aus einzelnen schleifenförmig gewundenen Einzelstegen mit Spalten dazwischen. Sie dient dem Fluiddurchlass in der zweiten Stellung, hält den Ventilkern in Sollstellung und bildet eine axial federnde und Schiefstellungen ausgleichende Haltestruktur.

In bestimmten erfindungsgemäßen Ausführungsformen weist der Ventilkörper einen - vorzugsweise pilzförmigen - Ventilkern auf. Letzterer weist einen - vorzugsweise steiferen - Kernbereich auf, der in beide axiale Bewegungsrichtungen als Bewegungsbegrenzung, d. h. an seiner Oberseite gegen die Folie und an seiner Unterseite gegen einen kassettenseitigen Stössel fungiert.

Die Anschläge werden nur berührt, wenn der spezifizierte Druck- und Volumenstrombereich verlassen wird. Eine Dichtringzone des Ventils weist in der ersten Stellung einen Ringspalt - von beispielsweise ca. 0,4 mm - zur sicheren Gassterilisation auf. In der zweiten Stellung dagegen dichtet sie - vorzugsweise unter Mindest-Vorspannung von ca. 0,4 mm - gegen einen - beispielsweise konischen oder flachen - Dichtsitz des Ventilsitzes ab.

In bestimmten erfindungsgemäßen Ausführungsformen weist die Rückschlagventilanordnung einen Ventilkörper mit einer Kappe auf oder besteht hieraus. Die Kappe ist vorzugsweise aus Thermoplast, vorzugsweise Polypropylen (kurz: PP), gefertigt.

In bestimmten erfindungsgemäßen Ausführungsformen weist der Ventilkörper, welcher einen Dichtring aufweist, eine Anzahl von Noppen auf, welche nach der Vormontage des Ventilkörpers mit der Kappe radial aus Durchbrüchen der Kappe nach außen ragen.

In bestimmten erfindungsgemäßen Ausführungsformen weisen sowohl der Ventilkörper als auch die Kappe Drainagestrukturen auf. Sie dienen zur optimalen Gas- und Dampf-Sterilisierbarkeit.

In bestimmten erfindungsgemäßen Ausführungsformen ist innerhalb des zylinderförmigen Abschnitts des Ventilsitzes der Kassette im Halteringbereich der Kassette eine als Durchmesserabsatz bezeichnete Stufe (stufenartige Durchmesserverjüngung) vorgesehen. Eine Aktivierung bzw. die zweite Stellung ist erreicht, wenn die radial überstehenden Noppen über den Durchmesserabsatz hinaus in den Ventilsitz der Kassette geschoben wurden und/oder wenn sich eine Ringstirnfläche der Kappe an die Ringstirnfläche des Ventilsitzes der Kassette bis zur flächigen Berührung angenähert hat.

In bestimmten erfindungsgemäßen Ausführungsformen ist die Kappe im Vergleich zum Ventilkörper steifer ausgestaltet.

In bestimmten erfindungsgemäßen Ausführungsformen kann die erfindungsgemäße Rückschlagventilanordnung zweiteilig sein.

In bestimmten erfindungsgemäßen Ausführungsformen kann die erfindungsgemäße Rückschlagventilanordnung flach dichten. Flach bedeutet in gewissen erfindungsgemäßen Ausführungsformen, dass die Dichtfläche - vorzugsweise ganz, im Wesentlich oder teilweise - eben und/oder parallel zu einer Folienebene ist. Die Dichtfläche kann in einigen erfindungsgemäßen Ausführungsformen bei flach dichtender Anordnung - vorzugsweise ganz oder im Wesentlichen - in einer Ebene liegen, die senkrecht zur Verschiebungsrichtung des Ventilkörpers liegt.

In bestimmten erfindungsgemäßen Ausführungsformen ist der Ventilsitz, gegen welchen der Ventilkörper und/oder die Kappe in der zweiten Stellung dichtet, im Dichtungsbereich konisch oder im Schnitt kegelförmig ausgestaltet.

In bestimmten erfindungsgemäßen Ausführungsformen hat der Ventilkörper die Form eines Napfes mit faltenbalgartigem Mantel, einen - optional weitgehend flachen, steif ausgelegten - Ventilboden und optional einen zentral und steif an den Ventilboden angebundenen Zuganker. Der Zuganker ist ausgestaltet, zur Verbindung zwischen Ventilkörper und Kappe, beispielsweise indem der Zuganker in eine passende zentrale Rastöffnung der Kappe eingerastet wird.

Die dem Ventilboden abgewandte Ringstirn des Faltenbalgmantels sitzt in bestimmten erfindungsgemäßen Ausführungsformen radial unter - vorzugsweise - leichter Vorspannung und axial unter größerer Vorspannung als radial in der Kappe. Dabei wird der Faltenbalgmantel entsprechend komprimiert, und die axiale Vorspannung wird über den eingerasteten Zuganker aufrechterhalten. Diese axiale Vorspannung kann hierin als Vorspannung des Rückschlagventils bezeichnet werden.

Die Kappe ist in bestimmten erfindungsgemäßen Ausführungsformen aus dem relativ steifen Werkstoff PP (Biege-E-Modul ca. 1750 N/mm) gefertigt.

Die Kappe ist in bestimmten erfindungsgemäßen Ausführungsformen mit radial ausfedernden Zungen ausgestaltet.

In bestimmten erfindungsgemäßen Ausführungsformen sind der Zuganker und der Ventilboden ausreichend steif ausgestaltet, so dass der Zuganker in der zweiten Stellung in alle Raumrichtungen berührungsfrei Abstand zur Bauumgebung in der Kappe hält.

In bestimmten erfindungsgemäßen Ausführungsformen weist die Kappe oder Rastkappe (beide Begriffe sind, soweit die Kappe in der jeweiligen Ausführungsform verrastbar ausgestaltet ist, als austauschbar zu verstehen) zusätzlich an einer unteren Ringstirn mehrere radial nach innen kragende oder ragende Haltenasen auf.

Die Kappe ist in bestimmten erfindungsgemäßen Ausführungsformen so konzipiert, dass sie in einem Vielfach-Auf-Zu-Spritzgiesswerkzeug mit jeweils zentralen Heisskanal-Anspritzungen hergestellt werden kann.

Der Ventilkörper weist in bestimmten erfindungsgemäßen Ausführungsformen einen zentralen konischen Dorn auf, welcher spielfrei in einem entsprechenden Konus der Kappe aufgenommen wird.

Eine Verbindungsmembran oder Membran erstreckt sich in bestimmten erfindungsgemäßen Ausführungsformen zwischen Konus und einem Halte-Dicht-Ring des Ventilkörpers.

Der Ventilkörper weist in bestimmten erfindungsgemäßen Ausführungsformen im nicht eingebauten Zustand eine leichte Verwölbung seiner mittig angeordneten Verbindungsmembran nach unten, d. h. im zusammengebauten Zustand in Richtung zum Ventilsitz der medizinischen Funktionsvorrichtung, auf.

Bei der Vormontage des Ventilkörpers in der Kappe wird diese Wölbung in bestimmten erfindungsgemäßen Ausführungsformen durch Einrasten eines Auflageflansches des Ventilkörpers bis zu deren Verzahnung mit den Haltenasen der Kappe bereits neutralisiert bzw. die Verbindungsmembran elastisch bereits so umgeformt, dass eine leichte Wölbung in die Gegenrichtung, also nach oben, d. h. weg vom Ventilsitz, zustande kommt. Die Wölbung in Gegenrichtung verstärkt sich in bestimmten erfindungsgemäßen Ausführungsformen ein weiteres Mal bei der Aktivierung bzw. beim Übergang in die zweite Stellung, und in bestimmten erfindungsgemäßen Ausführungsformen ein weiteres Mal bei Durchströmung mit Behandlungsfluiden.

Der Halte-Dicht-Ring ist in bestimmten erfindungsgemäßen Ausführungsformen im Verhältnis zur Verbindungsmembran kompakter und/oder dickwandiger ausgeführt.

Die Rückschlagventilanordnung hat in bestimmten erfindungsgemäßen Ausführungsformen die Gestalt eines Dornteller-in-Kappe.

In bestimmten erfindungsgemäßen Ausführungsformen weist der Ventilsitz keine Hinterschnitte auf.

In bestimmten erfindungsgemäßen Ausführungsformen hat die Kappe die Form eines Gewölbes mit mehreren radial nach außen und axial nach oben offenen Durchbrüchen. In diesen Durchbrüchen sind Rastzungen angeordnet, die sich radial nach innen biegen lassen. Die Rastzungen verschließen die Durchbrüche nur anteilig, beispielsweise zu ca. 30 %.

Räumliche Angaben wie "oben", "unten", usw. beziehen sich im Zweifel auf die Darstellung, wie sie den hier beigefügten Figuren zu entnehmen ist.

In bestimmten erfindungsgemäßen Ausführungsformen sind die Anzahl der Durchbrüche und die Anzahl der Rastzungen bevorzugt jeweils ungerade.

In bestimmten erfindungsgemäßen Ausführungsformen weist die Kappe eine umlaufende, vorzugsweise scharfe Kante auf, die sich sowohl bei den Stützbögen als auch bei den biegsamen Rastzungen vorzugsweise auf gleicher Höhe befindet und dabei die Haupttrennebene des Spritzgusswerkzeugs bildet.

In bestimmten erfindungsgemäßen Ausführungsformen bildet eine obere Kappen-Ringstirn den höchsten Abschnitt der Gewölbekonstruktion und stellt durch die Folie hindurch die mechanische Schnittstelle zur Einleitung von Aktivierungskraft, Aktivierungsweg und Haltekraft durch die Aktor-Sensor-Einheit der Behandlungsmaschine dar. Sie stellt eine durch Strukturierungsrillen unterbrochene ebene Ringstirnfläche dar.

In bestimmten erfindungsgemäßen Ausführungsformen weist die Kappe sowohl auf der oberen Ringstirn als auch im Bereich der Konus-Aufnahme zahlreiche Strukturierungen wie Strukturierungsrillen, Nuten und Rücksprünge auf.

Damit das Bauteil eine symmetrische und beim Abkühlen des Thermoplasten während der Fertigung verzugsunanfällige Form behält, sind in bestimmten erfindungsgemäßen Ausführungsformen die äußeren und inneren Strukturierungen in einer konzentrischen und gleichzahligen oder geradzahlig geteilten Anzahl in Relation zur Anzahl der Durchbrüche oder Rastzungen angeordnet.

In bestimmten erfindungsgemäßen Ausführungsformen weist die Kappe nicht nur die Gewölbestruktur, sondern auch vertikale Zylinderwände und konische, insbesondere steilkonische, Umlaufwände, vorzugsweise innerhalb und außerhalb einer Kappen-Zentrier-Rille, auf.

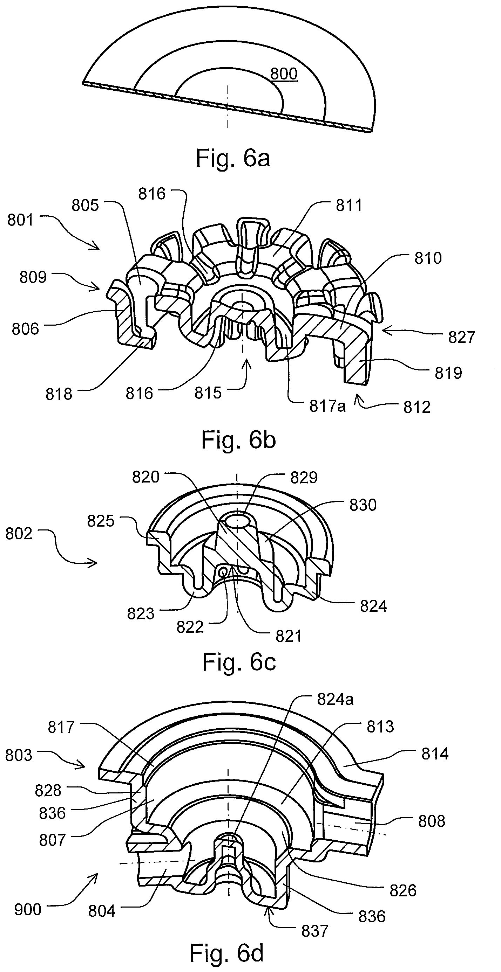

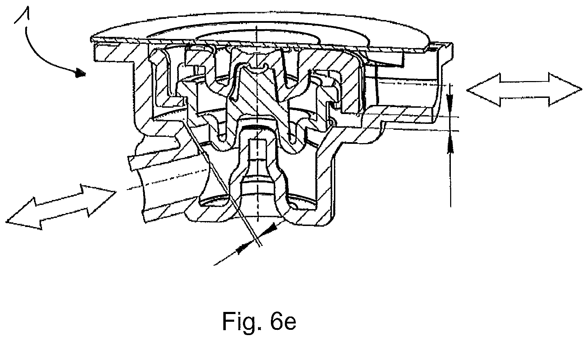

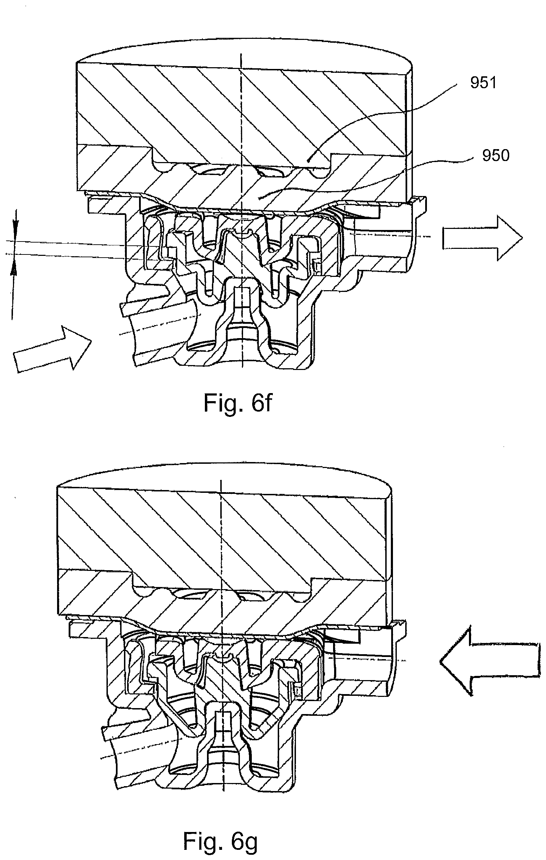

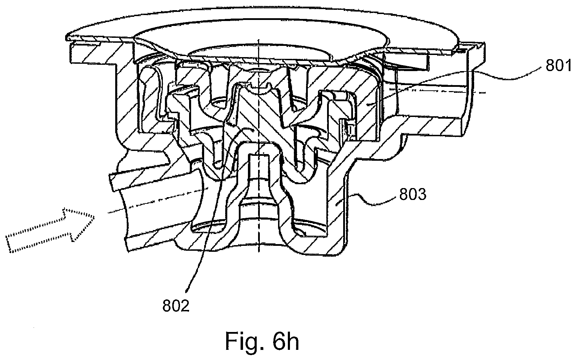

Der Ventilkörper hat in bestimmten erfindungsgemäßen Ausführungsformen eine rollbalgähnliche Form und umfasst einen Zentrierkonus, eine Anschlagstirn, wenigstens zwei Hilfszentrierhöcker, eine federnde Verbindungsmembran, einen Dichtring und einen Haltering oder besteht aus diesen, wobei die vorstehenden Begriffe mit Bezug auf Fig. 6 unten erläutert sind.

In bestimmten erfindungsgemäßen Ausführungsformen sind, um die Einführmöglichkeit zusätzlich zu steigern, sowohl die untere Ringstirn der Kappe als auch die Ventilsitzkante am Foliensteg mit Rundungen versehen, die als zusätzliche Einführschrägen fungieren.

In bestimmten erfindungsgemäßen Ausführungsformen haben die Rastzungen einen gegenüber den äußeren Zylinderwänden der Kappe um z. B. 0,4 mm größeren Durchmesser.

In bestimmten erfindungsgemäßen Ausführungsformen ist der Ventilkörper in der Kappe vorgespannt kraft- und formschlüssig und axial spielfrei eingebaut.

Einige oder alle erfindungsgemäßen Ausführungsformen können einen oder mehrere der oben oder im Folgenden genannten Vorteile aufweisen.

Bei manchen erfindungsgemäßen Ausführungsformen kann die Aktivierung, d. h. die Rückschlagfunktion vorteilhaft auch nach dem Abrüsten des Disposables erhalten bleiben. Damit kann das Austreten von Flüssigkeit auch beim Abrüsten an den drei genannten Stellen mittels der erfindungsgemäßen, remanent aktivierten Rückschlagventile verhindert werden. Hierdurch können am Disposable zwei Schlauchklemmen und ein zu ihrer Betätigung erforderliche Tätigkeit des Anwenders eingespart werden. Weiterhin bedarf es vorteilhaft keiner Verschlusshülse im Substituatkonnektor (siehe hierzu das Bezugszeichen 41 in Fig. 1 der WO 2010/121819 A1) und eines dazugehörigen Arbeitshubs der Maschine, um einen Schutz vor Kontamination durch aus der Kassette austretende Flüssigkeiten zu bieten.

Die erfindungsgemäßen Rückschlagventile können vorteilhaft die erforderliche Robustheit gegenüber vorzeitiger Aktivierung in der Produktion vor der Sterilisation aufbieten.

Des Weiteren können erfindungsgemäßen Ausführungsformen Vorteile bei der automatischen Herstellung, der sicheren Sterilisation und der Qualitätssteigerung der Rückschlagventilfunktionen bieten. Hierzu tragen großlumige Drainagestrukturen zu glatten Folien und glatten Spritzgußwänden und geringe Anteile an flächigen Komponentenberührungen bei, ferner ein sicheres Verbleiben in der offenen Initialisierung, also der ersten Stellung, bis zum Abschluss der Gassterilisation, geringe und definierte Eigenschaftsänderungen durch die Gassterilisation insbesondere des Öffnungsdrucks, die Minimierung von Eigenschaftsänderungen der eingebauten Rückschlagventile durch mechanische, thermische oder bestrahlungsinduzierte Belastung während der Lagerung und des Transports, welche den Vorgang der sicheren und präzisen Aktivierung im Rahmen des Aufrüstvorgangs beeinträchtigen könnten, Vermeidung des bisher tolerierten Überstandes der folienseitigen Stirnflächen der Rückschlagventile über die Folienebene der Kassette (Rückschlagventile verursachen lokal Beulen in der Folie) zur Gewährleistung einer sicher herstellbaren Folienschweißnaht, ausgehend von einer durchgehend eben auf der Kassette aufliegenden Folie, toleranzen-tolerantere Gestaltung der Einzelteile und ihres Zusammenwirkens sowohl bei der Montage wie im Zusammenwirken mit der Behandlungsmaschine bei gleichzeitig hoher Reproduzierbarkeit der Dichtheit, des Öffnungsdrucks und der Durchfluss-Druckabfall-Kennlinie und dadurch sichere Funktion auch bei Großserienproduktion und vielen bzw. gealterten Behandlungsmaschinen im Markt, Erhöhung der Reproduzierbarkeit des Durchlass-Öffnungsdrucks, Verringerung des Druckabfalls im Durchlassbereich, also Minimierung des Druckabfalls bei jedem Durchlass-Volumenstrom, Verflachung der Volumenstrom-Druckabfall-Durchfluss-Kennlinie, also Minimierung der Zunahme des Druckabfalls mit Zunahme des Durchlass-Volumenstroms, Erhöhung der Fein- und Langzeit-Intrusionsdichtheit unterhalb des Durchlass-Öffnungsdrucks, insbesondere Dichtheit gegen Intrusion von Luft, Verminderung der Empfindlichkeit gegen Undichtheit bei Anwesenheit von Verschmutzungs-Partikeln und Verbesserung der Selbstreinigungsfähigkeit, weiterhin sichere und dichte Abdichtung im Sperrbereich bis zum Maximaldruck, und Vermeidung von Schwingungen und Geräuschemissionen.

Zu den weiteren Vorteilen zählen:
∘ das geringe Risiko der Fehlfunktion nach Aktivierung;
∘ sicheres Funktionieren auch bei maximal möglichem axialem Aktivierungshub (an der Folie gemessen) von zirka 1,2 mm, was von der Kassettenkonstruktion und der Behandlungsvorrichtung vorgegeben ist;
∘ Verbesserung der fluidischen Kennwerte ist erzielbar, da diesbezüglich verbesserte Bauform und kleineren Toleranzen bei Bauteilen und Aktivierung erzielbar;
∘ reversible Aktivierung ist während der Herstellung zur Funktionsprüfung durchführbar;
∘ kein Problem einer ungleichmäßigen Spritzguss-Herstellung mit Bindenähten und damit verbundener Undichtheit;
∘ hinreichend gleichbleibende Geometrie nach der Dampfsterilisation;
∘ vernachlässigbare Relaxation während der vorgespannten aktivierten Stellung, welche die Ventilvorspannung im Gebrauch herabsetzten und damit die Parameter Öffnungsdruck und Durchlass-Druckabfall beeinflussen könnte;
∘ hohe Toleranzüberbrückung durch Materialkombination Elastomer-Thermoplast und durch Noppengeometrien, dadurch preisgünstige und sichere Herstellung mit großen zulässigen Maßtoleranzen;
∘ sichere axiale Kraftbegrenzung, da vollständig aus Elastomer und durch Fußrippen axial nachgiebig;
∘ gute Gaststerilisierbarkeit durch großlumige Drainagen und durch Balligkeit der Klemm-Zentrierrippen geringe Verpressungsflächen zwischen Ventilkörper und Ventilsitz;
∘ Ansprechdruck und Druck-Volumenstrom-Kennlinie sind nur abhängig von den gewählten Materialen, den gewählten Wandstärken und Durchmessern und von der Herstelltoleranz der Abmessungen und der Materialeigenschaften, nicht jedoch von der Größe des Aktivierungshubes, sofern der effektive Aktivierungshub im geplanten Bereich (hier 1,2 bis 1,8 mm) liegt;
∘ keine Hinterschnitte und damit keine Entformungsprobleme bei der Gestaltung der Ventilsitze in der Kassette;
∘ weiterhin einteiliges Rückschlagventil mit geringem Risiko der Fehlfunktion nach Aktivierung;
∘ einfacher Montagevorgang;
∘ guter Toleranzausgleich gegen axiale Einbau-Schiefstellungen;
∘ Entkopplung des Aktivierungswegs und damit der Ventilvorspannung von den Maschinentoleranzen;
∘ deutliche Verbesserung der Reproduzierbarkeit von Öffnungsdruck und Druck-Volumenstrom-Kennlinie;
∘ Verflachung der Druck-Volumenstrom-Kennlinie; dadurch sind geringere Druckverluste und geringerer Auslege-Öffnungsdruck realisierbar;
∘ ergonomische Vorteile durch geringere Spritzenbetätigungskraft bei Medikamenteninfusion;
∘ geringere Hämolyse bei Nutzung für Blutinfusion;
∘ einfache Vormontage des Ventils in der Rastkappe oder Kappe bei hoher Reproduzierbarkeit der radialen Zentrierung;
∘ Möglichkeit, die Ventilbaugruppe bei der Herstellung nach Einbau der Kassettenfolie auf Funktionsfähigkeit zu testen;
∘ durch die Faltenbalgform des Ventils lässt sich eine linearere Federkennlinie realisieren als beispielsweise mit der einfachen Pilzform ;
∘ der Dichtringbereich des Ventilelements befindet sich im Bezug auf die Fließverhältnisse bei bevorzugter Herstellung als LSR-Spritzgussteil (Liquid Silicon Rubber) nicht am Ende des Fließweges (wie etwa bei der einfachen Pilzform), dadurch Vermeidung von Bindenähten und damit von Undichtheit verursachenden Formfehlern;
∘ sehr materialarme, kostengünstige Realisierung des Rückschlagventils (0,5 Euro-Cent);
∘ drehpositionierfreie leicht automatisierbare Montage der Ventilbaugruppe;
∘ gutes Zentrierverhalten des Dichtringbereichs;
∘ sichere Vermeidung von Reibung zwischen Ventil und Kappe bei Aktivierung und Durchströmung;
∘ axial besonders kleinbauende Ventilanordnung mit dadurch verringerbaren Strömungs-Toträumen;
∘ Vorteile im Kennlinien- und Toleranzverhalten wie die anderen vorgespannten Ventilbauarten mit Kappe;
∘ gasoffene Initialstellung zur Gewährleistung sicherer Gas-Sterilisationsverfahren;
∘ robuste Initialstellung mit hoher Auslöse-Schwellkraft trotz geringem Aktivierungsweg;
∘ Vermeidung des Austretens unerwünschter Fluide nach Abrüstung durch remanente Aktivierung;
∘ sichere remanente Aktivierung während Aufrüstung mit sicherem Stellungserhalt nach Abrüstung durch Reibschluss und großen Vorspannweg des aktivierten Ventils;
∘ harte abdeckende Kappe kapselt das weiche Ventil gegen mechanische Eingriffe ab;
∘ neuer Rastzungen-Keil-Durchmesserabsatz-Mechanismus für robuste gasoffene Initialstellung und definierte stark erhöhte Auslöse-Schwellkraft;
∘ extrem geringer Aktivierungsweg im Verhältnis zum Ventildurchmesser ermöglicht maschinenseitig passive Aktivierungsaktoren bei flacher Folien-Ausgangslage und geringer Foliendehnbeanspruchung;
∘ neuer verkantungssicherer Aktivierungs- bzw. Gleitlagermechanismus unter bewusster Einbeziehung möglicher Verkippungen in das reguläre Verschiebeverhalten;
∘ besondere geometrische Anordnungen mit Reduktion der Toleranzketten;
∘ verminderte Toleranzen durch präziser herstellbare kleinbauende, funktionsbestimmende Geometrien;
∘ Verringerung der Ventiltoleranzen durch gegenseitige geometrische Kalibrierung zusammengebauter Komponenten;
∘ Nutzung der thermischen und zeitlichen Materialspannungsabbaus durch Sterilisation und Lagerung, um die Aktivierungs-Schwellkraft zu kalibrieren und bei der Behandlung herabzusetzen, während der Ventil-Herstellung jedoch möglichst hoch anzusetzen;
∘ Entkopplung der Positionstoleranzen des Ventils zum Ventilsitz von den eingeleiteten Kräften und Wegen der Behandlungsmaschine durch Zwischenschaltung der Kappe;
∘ Prinzip der Vorspannung führt zu größerem Vorspannweg und in Folge zu genaueren und druckverlustärmeren und damit auch behandlungsfluidschonenderen (geringere Scherspannungen bei gleichem Volumenstrom) Ventileigenschaften;
∘ konische oder kugelkalottenförmige Ventil-Dichtsitze führen zu toleranzenärmeren Ventileigenschaften, zu Dichtsteigerung bei Stillstand, zu Selbstreinigungsfähigkeiten, zu höheren Dichtfähigkeiten bei Fremdkörpern und bei lokalen Materialfehlern;
∘ global steife und lokal spitze und weiche Ventildichtringgeometrien führen zu höheren Dichtfähigkeiten bei Fremdkörpern und bei lokalen Materialfehlern;
∘ extrem günstige Herstellkosten der Einzelkomponenten durch Auf-Zu-Entformungsprinzipien und minimierte Spritzguss-Zykluszeiten durch gleichmäßige Wandstärken und geringen Materialverbrauch und wegen geringer Dehnbeanspruchung Einsatzmöglichkeit einer relativ wenig elastomerhaltigen und deshalb preisgünstigen Folie;
∘ spezifisch genauere Komponentengeometrie durch rotationssymmetrische, spezifisch steife Geometrie und zentrale Anspritzung;
∘ keine oder kaum Lufteinschlüsse und Bindenähte in der Dichtringzone durch geometrische Verlagerung weg vom Fließwegende;
∘ vollständig automatisierbares Herstell- und Prüfkonzept konzeptionell in die Teilegeometrien eingeplant unter Berücksichtigungen aller Teile- und Handlingstoleranzen mit besonders guten Selbstzentriereigenschaften und Freiheit von Drehpositionierungen aller Komponenten;
∘ vollständige fluidische Prüfung des Ventils im fertig eingebauten Zustand durch reversible Aktivierung umsetzbar;
∘ selbstkompensatorische Eigenschaften gegen Kennlinien-Relaxation im Betrieb;
∘ Nutzung rollbalgspezifischer Verformungseigenschaften zur Verflachung der Druckverlust-Volumenstrom-Kennlinie;
∘ optimierte Drainage- und Sterilisations-Strukturierungen der thermoplastischen Kappe als funktionale Verbesserung im Vergleich zu Strukturierungen der elastomeren Ventilkomponente;
∘ konische Zentrierungen mit großem Öffnungswinkel, Strukturierungen in Axialrichtung, Matterodierungen und axialen Anschlagzonen bei der Kappe für optimierte Zusammenbaufunktionalität mit dem weichen klebrigen Elastomermaterial des Ventils;
∘ Möglichkeit den Öffnungsdruck wegen der höheren Reproduzierbarkeit bei gleichem Sicherheitsniveau auf einen niedrigeren Wert zu setzen und daher ergonomischere Zuspritzung von Behandlungsfluiden, geringeres materialschonenderes Druckniveau von Zuführpumpen oder geringere Hämolyserate bei Blutrückgabe durch das Rückschlagventil;
∘ viel- und/oder ungeradzahlige gleichmäßige Teilungsanordnung von Durchbrüchen und Rastzungen, die eine druckverlustarme und gleichmäßige Durchströmung der Ventilanordnung ohne Drehorientierung auch bei mehreren vom Ventilsitz abgehenden Fluidkanälen mit guter Zentrierfähigkeit ermöglichen;
∘ funktionstechnische Nutzung des Entformungs-Trenngrates für Funktionalitäten des Ventils wie Reibung bei Axialverschiebung und verkantungsunempfindliche Verkippung mit Wegfall der sonst zumeist gegebenen Notwendigkeit, das Spritzgussteil gratarm herstellen zu müssen;
∘ großflächige Aktivierungs-Ringfläche aus hartem ThermoplastMaterial reduziert die Aktivierungs-Verpress-Spannungen auf den Aktivierungshöcker der Maschine und reduziert Ungenauigkeiten der Aktivierungsweg-Übertragung durch elastische Nachgiebigkeiten an Maschine und Ventilbauteilen;
∘ günstige Formgebung in Schwerpunkt und Formasymmetrie bei Kappe und Ventil zur sicheren Vibrationsförderung und Lagesortierung bei Teilebereitstellung und - vorpositionierung;
∘ konisch runde zentrische Geometrien bei Kappe und Ventil erlauben eine Ansaugung und präzise Weiter- und Endzentrierung mit röhrenförmigen Vakuum-Greifern und runden Aufnahme-Bohrungen;
∘ Einsatzmöglichkeit einer besonders einfachen passiv wirksamen Aktivierungsvorrichtung auf der Maschinenseite mit kostenneutraler Integration einer progressiv wirkenden Aktivierungs-Höcker-Federung in die hermetisch abgekapselte Rückseite der elastomeren Aktor-Sensor-Matte;
∘ Einsatzmöglichkeit einer ebenfalls abgekapselt eingebauten diskret mit technischen Federn aufgebauten Aktivierungsvorrichtung im Raum hinter der Aktor-Sensor-Matte;
∘ Einsatzmöglichkeit einer aktiv antreibbaren Aktivierungseinheit abgekapselt hinter der Aktor-Sensor-Matte mit der Möglichkeit, vollständige Funktionstests der Ventilanordnung durch reversible Aktivierung noch unmittelbar vor der Behandlung durchführen zu können und weiterhin außer der Rückschlagventilfunktion auch die Funktion beidseitig offener Strömungswege verfahrenstechnisch nutzen zu können;
∘ Nutzung der Unterschiede von Haft- und Gleitreibung im Aktivierungs-Mechanismus der Kappe, um eine hohe Aktivierungs-Schwellkraft mit geringer Weiterschiebe-Kraft nach Überwindung der Rast-Barriere zu erhalten;
∘ Nutzung von balligen oder spitzen Noppen oder Wellen-Strukturen in der Aktor-Sensor-Matte, um gezielte progressive Kraft-Weg-Kennwerte zu erhalten, die eine hohe Aktivierungs-Schwellkraft mit geringer Restkraft in aktivierter Stellung verbinden können;
∘ Ventil-Öffnung rein durch Biegevorgänge im elastomeren Ventil, dadurch weitgehender Wegfall von Hysteresen und Reproduzierbarkeits-Schwankungen;
∘ geringe Zusetz-Neigung durch gleichmäßiges und vollständiges Abheben des Dichtrings beim Öffnen des Ventils unter Vermeidung von Strömungstotzonen und Fluid-Verweilzeiten an nicht abhebenden Zonen des Dichtrings; und
∘ geringe Empfindlichkeit zur Verursachung von Schwingungen und Geräuschen, da der Ventil-Dichtring gleichmäßig abhebt und somit im Wesentlichen nur eine Grundschwingungsform zulässt.
In bestimmten erfindungsgemäßen Ausführungsformen kann auch das Ventil-Spritzgießwerkzeug vorteilhaft nach einer günstigen Auf-Zu-Konzeption gebaut werden, wobei die erforderliche Formnest-Zahl wegen der längeren spezifischen Zykluszeit zur Vernetzung des Flüssigsilikons etwa um den Faktor 2 höher auszulegen ist als beim Kappenwerkzeug. Durch die einfache im Wesentlichen rotationssymmetrische Formgebung, durch die plan abdichtbare, formversatz- und formgratunkritische Formtrennung an der unteren Umlaufkante des Halterings und durch das sehr kleine Bauvolumen von zirka 110 mm^3 ist es möglich, das Ventil äußerst günstig herzustellen. Da LSR-Bauteile in der Regel am wirtschaftlichsten durch lateral ansetzende rotierende Spachtel aus den auswerferseitigen Formnestern ausgefegt werden, ist das Ventil ganz auf die optimale Herstellbarkeit hin gestaltet und nutzt hierbei den Zentrierkonus als zentralen Anspritzpunkt und bei der Entformung als aus der auswerferseiten Formhälfte herausstehenden Ausfegnippel. Die Rückschlagventilbauformen der erfindungsgemäßen Funktionsvorrichtung weisen die Eigenschaft auf, zentral angespritzt und im Wesentlichen rotationssymmetrisch gestaltet zu sein, was für eine verzugsarme Formgestalt von Vorteil ist.

Vorteilhaft ist auch, dass kein großer Anteil ihres Volumens zentrumsnah angeordnet ist (Pilzfuß-Bereich), während der Anteil ihres Volumens in der Nähe des Dichtrings relativ groß ist und sich dabei nicht in unmittelbarer Nähe zum FließwegEnde und zur entlüftenden Formtrennung befindet. Dies führt dazu, dass der flüssige Spritzgießwerkstoff bei der Füllung des Dichtring-Bereichs nicht bereits kurz vor Beendigung der Formfüllung ist, die Fließgeschwindigkeiten nicht zum Erliegen kommen und Luftblasen und Bindenähte mehr weitergeschoben werden. Man hat also nicht das Problem, Lufteinschlüsse und Bindenähte mit den damit verbundenen geometrischen Ungenauigkeiten an der Oberfläche ausgerechnet am Dichtring zu erhalten und damit die Dichtfähigkeit des Ventils am ringförmigen Bereich der Berührung mit der Dichtfläche am Ventilsitz herabzusetzen.

Das Rückschlagventil der erfindungsgemäßen Funktionsvorrichtung weist in bestimmten erfindungsgemäßen Ausführungsformen eine Form auf, welche bei seiner Herstellung ein Befüllen der Spritzform besonders begünstigt: das zentral eingespritzte Flüssig-Elastomer prallt zunächst gegen die Anschlagstirn und wird schroff in die dünnwandige Wellenform der Federmembran umgelenkt, fließt nach erneuter Umlenkung durch den Dichtring und weiter zum relativ voluminösen Haltering, wo sich die Formtrennung mit Entlüftung und Fließwegende befinden. Der Dichtring wird also mit gut homogenisierter Spritzgießmasse bei relativ hoher Fließgeschwindigkeit durchströmt, und damit werden Lufteinschlüsse und Bindenähte (teilweise erstarrte Fließfronten) weitergeschoben. Somit erhält man einen Dichtring mit besonders genauer und für eine Abdichtung geeigneter Oberfläche.

Wegen der geringen Steifigkeit von elastomeren Bauteilen (hier zirka 40 bis 70 Shore A) im Verhältnis zur relativ hohen Steifigkeit der Kappe, sind in bestimmten erfindungsgemäßen Ausführungsformen Strukturierungen zur Zugänglichkeit von Sterilisationsgasen, zur Luftabfuhr und Reibminderung beim Zusammenbau vorteilhafter Weise eher in der Kappenkomponente als in der Ventilkomponente angeordnet. Man braucht in der Kappe weniger tiefe Strukturen, um im verpressten zusammengebauten Zustand gleich große offen bleibende Strukturen zu erhalten als bei Anordnung entsprechender Strukturen in der Ventilkomponente. Dies ergibt sich, da sich am Elastomer die erhabenen und eingeprägten Strukturen abplatten und dadurch die ungewünschten direkten Berührflächen zwischen den Thermoplast- und den Elastomerkomponenten erhöhen und gleichzeitig die gewünschten gasführenden Drainagerillen im Querschnitt abnehmen. Zusätzlich kommt es der fehlerarmen Herstellbarkeit der Oberflächen des Elastomerteils zugute, wenn es keine schwer ausspritzbaren und schwer entlüftbaren feinen Strukturen gibt, was es bei der Herstellung der Kappe nicht zu beachten gilt, da hier kleine Fehlstellen in der Oberfläche keinen Einfluss auf die Funktion haben.

Weitere mögliche Vorteile betreffen insbesondere die Toleranzenoptimierungen. Im Einzelnen:
1. Durch die Vorspannung des Ventils in der Kappe passt sich das Ventil in mehreren axialen Abmessungen an die Kappe an und richtet sich auch im Bezug auf Planlauf, Radialzentrierung und Achsparallelität nach der steiferen und genauer herstellbaren Kappe, womit bereits einige Maßtoleranzen des Ventils an Bedeutung verlieren oder gar bedeutungslos werden.
2. Dadurch, dass für die Stellung "aktiviert" nur mehr die Tatsache eine Rolle spielt, dass die Kappe mit ihrer unteren Ringstirn an der Ventilsitz-Ringauflage plan anliegt, ist es bedeutungslos, welchen Weg die Aktor-Sensorplatte der Behandlungsmaschine zur Verschiebung der Kappe in den Aktivierungshöcker einleitet, so lange der Weg mindestens so groß ist, dass es bei der Kappe zu einer planen Anlage kommt. Dies ist ein markanter Unterschied zu den meisten, weich bauenden, bekannten Ventilbauarten mit proportionaler Abhängigkeit des Aktivierungswegs des Ventils vom Aktivierungsweg der Maschine. Man erhält also eine annähernd vollständige Entkopplung zwischen den Aktivierungswegen des Ventils und der Maschinen-Aktivierungsaktorik.
3. Dadurch, dass die Kappe eine um Größenordnungen steifere Komponente darstellt als der elastomere Ventilkörper, sind auch die axialen Eigenverformungen unter den eingeleiteten Aktivierungskräften entsprechend geringer. Hierdurch fällt die axiale Positionsunsicherheit des Zentrierkonus der Kappe und damit des Zentralbereichs des Ventils um mehr als eine Größenordnung kleiner aus (0,02 bis 0,04 mm) als bei der bisherigen kappenlosen Bauart, bei der die Eindringtiefe und die Axialkraft des Aktivierungshöckers der Behandlungsmaschine direkten Einfluss auf die axiale Verlagerung des Zentralbereichs des Ventils und damit die Betriebsvorspannung des aktivierten Ventils genommen hatten. Man erhält somit eine annähernd vollständige Entkopplung zwischen den Vorspannkräften des Ventils in Aktivierungsposition und den Aktivierungskräften der Maschinen-Aktivierungsaktorik.
4. Die geometrisch-mechanische Kette der beeinflussenden Toleranzen schließt nur mehr einen kleinen Teil der bisherigen Abmessungen ein, nämlich die Abmessungen des Ventils und der unmittelbar um dieses herum an der Ventileinspannung beteiligten Topologien des Ventilsitzes und der Kappeninnenseite. Da all diese Topologien nur mehr kleine Abmessungen im Bereich von 0,4 bis 3,6 mm in Z-Richtung haben, sind auch die erzielbaren Herstelltoleranzen der Spritzgusstopologien entsprechend klein und liegen im Bereich von weniger als +/- 0,03 mm. Man erhält also signifikante Verkürzungen der Toleranzketten und zugleich engere Einzel-Toleranzen.
5. Durch die Plananlage der beiden obengenannten Ringflächen unter einer bleibenden Restkraft von typischerweise 5 bis 20 N reduzieren sich die Planlaufabweichungen beider Ringflächen gegenseitig, da sie sich gegenseitig elastisch-plastisch einebnen. Dies ist ein typischer Vorgang, falls die grundsätzlich von Winkelverzügen betroffenen Spritzgusskomponenten axialen Verpressungen gegen richtende Gegenflächen ausgesetzt werden. Man erhält so eine Genauigkeitssteigerung durch gegenseitige Gestaltoptimierungen der verbauten Komponenten.
6. Die Federmembran des Ventils hat eine rollbalgähnliche Gestalt. Die Gestaltänderungsvorgänge bei der axialen Auslenkung des Ventils sind aber nicht gleichzusetzen mit einem echten Rollbalg, da die zylindrischen Abwälzflächen zur Vermeidung von Bewegungsreibung fehlen. Die rollbalgähnliche Gestalt hat aber spezifische Vorteile gegenüber einer flachen Federmembran:
6a. Bei gleicher axialer Vorspannung ist die Winkelnachgiebigkeit auf dem Niveau des Dichtrings deutlich erhöht, was zu gleichmäßigerer Dichtverpressung und damit engerer Streuung des Öffnungsdrucks bei radialem und winkligem Versatz der Ventilsymmetrieachse zur Ventilsitzsymmetrieachse führt.
6b. Durch die relative Steifigkeit des Halterings in Verbindung mit dem weitgehend vertikalen Wandabschnitt zwischen Haltering und Dichtring ergibt sich eine global spezifisch steife Dichtringzone, die sich durch die Rollmembran eher als Ganzes verlagert und sich so dem Dichtring des Ventilsitzes winkel-, radial- und axialverlagert anpasst. Lokal, also im Bezug auf die örtliche Verpressung des elastomeren Ventildichtrings auf den harten Ventilsitzdichtring, wird die dichtende Anpassung beider Oberflächen dadurch verstärkt, dass der Ventildichtring relativ scharfkantig baut (spitzes Element einer Dichtpress-Paarung) und sich über dessen geringe Härte von ca. 30 bis 70 Shore A lokal am Ventilsitzdichtring abplattet. Dabei entsteht lokal eine hohe Flächenpressung, welche das entscheidende Kriterium einer lokal wirksamen dichtenden Überbrückung lokaler Unregelmäßigkeiten der beiden Dichtflächen oder lokal in der Dichtzone vorliegender Fremdkörper darstellt. Bei den meisten der bisher vorgeschlagenen Ventilbauformen ist die Ventildichtringzone dünnwandig und global oder insgesamt wenig steif. Dabei führen Unregelmäßigkeiten und Fremdkörper zu einer globalen Umformung der Dichtringzone und nicht zu einer lokalen Überbrückung und Einkapselung, weshalb die Dichtwirkung durch lokale Lückenbildung herabgesetzt wird. Das Ventil der erfindungsgemäßen Funktionsvorrichtung dichtet also reproduzierbarer und besser unter dem Einfluss lokaler Oberflächenfehler und in der Dichtringzone vorhandener Fremdkörper.
6c. Die rollbalgähnliche Gestalt hat auch einen Einfluss auf die Linearität der Federkennlinie der axialen Dichtringverlagerung. Rollbälge weisen ab einer bestimmten axialen Mindestauslenkung eine konstant bleibende oder sogar zurückgehende Kraft bei beliebiger weiterer Auslenkung auf. Die Mindestauslenkung ist dann erreicht, wenn der Biegspannungszustand sich bei weiterer Auslenkung nicht mehr ändert, der Rollbalg also eine konstant ähnlich bleibende Form angenommen hat. Der dazu erforderliche Auslenkungs-Axialweg ist bei einem echten Rollbalg ca. 3 bis 5-mal so hoch wie die Rollbalgstärke. Durch die Vorspannung des Kappen-Ventils kann ein solcher Weg eingeprägt werden, was bei vorspannungslosen Bauarten durch den beschränkten Aktivierungsweg von 0,8 mm nicht möglich ist, da von diesem Weg für die axiale Vorspannung nach Abzug des Mindest-Sterilisations-Spalts und aufsummierbarer Toleranzen nur mehr ca. 0,4 mm übrig bleiben, was bereits weniger ist als die vorhandene Rollbalgstärke. Die rollbalgtypische Eigenschaft der Begrenzung der Axialkraft macht es also möglich, Ventile so auszulegen, dass die Kraft-Weg-Kennlinie annähernd waagerecht verläuft und damit die Toleranzempfindlichkeit des Öffnungsdrucks und des Strömungswiderstands noch einmal weiter herabgesetzt werden können.
7. Durch die Vorspannung des Ventils in der Kappe und die Aufhebung dieser Vorspannung und weitere Vorspannung in der zweiten oder der aktivierten Stellung kann das neue Ventil mit deutlich höherem Weg vorgespannt werden als Ventile ohne Kappe und ohne Vorspannung; also lassen sich zum Erzielen gleicher Vorspannung in der zweiten Stellung nachgiebigere Federmembrangeometrien mit flacherer FederKennlinie verwenden, die zu flacherer Druckverlust-Volumenstromkennlinien und damit bei gleicher Aktivierweg-Ungenauigkeit zu kleineren Unsicherheiten des Öffnungsdrucks wie der Druckverluste und Druckverlust-Ungenauigkeiten führen.
8. Da die Kappenkonzeption aber wie oben erklärt zu einer weitgehenden Entkopplung des Ventils von der Maschinenaktorik führt und zudem durch geschickte Bauteilanordnung die geometrischen Toleranzen abnehmen, werden die Toleranzen der vorstehend genannten Kennlinien-Werte weiter verkleinert.
9. Durch die toleranzarme zweite, aktivierte Stellung in Verbindung mit der flacheren Ventil-Federkennlinie und der stärkeren Vorspannung erreicht man eine besonders sichere remanente Aktivierung des Ventils nach dem Abrüsten des Disposables. Die reibschlüssig in der Aktiviert-Stellung gehaltene Kappe setzt sich selbst dann nicht in Bewegung, wenn im Disposable ein Überdruck von bis zu 0,6 bar auftritt, was unter regulären Abrüstbedingungen in der Praxis nicht vorkommen kann, da die Gaskompliance des entleerten Disposable den Aufbau eines solchen Drucks nicht zulässt.

Mit der Keilwirkung und mit der höheren Dichtpressung einher geht in bestimmten erfindungsgemäßen Ausführungsformen auch ein höherer Selbstreinigungseffekt des Ventils beim Öffnen und Schließen, eine besonders nützliche Eigenschaft, denn durch die keilförmige Anordnung des Ventilsitz-Dichtrings überlagern sich beim Abheben des Ventils dort eine normale und eine tangentiale Bewegung, wobei die tangentiale Bewegung den Reinigungseffekt, aber bis zum Abheben des Ventils auch den hier gewünschten Hystereseeffekt (höherer Öffnungsdruck) nach längerer Dichtzeit verursachen. Der bevorzugt konische Ventil-Dichtsitz hat, wie weiter oben bereits angedeutet, günstigere Winkeltoleranzeigenschaften als der flache Ventilsitz, da er der Form einer Kugelkalotte näher kommt. In einer Ideal-Ausführung kann der Ventil-Dichtsitz die Form einer Kugelkalotte einnehmen, welcher man genau den Radius aufprägt, die einer toleranzbedingten Pendel-Schiefstellung der Ventilachse zur Ventilsitzachse entspricht: dann benötigt der Ventil-Dichtring nur mehr ein Minimum an globaler elastischer Verformung, sich an den Ventil-Dichtsitzring anzupassen.

Allerdings ist die Auslegung der Vorspannungsverhältnisse schwieriger, da die Konuswände keine konstante Steigung mehr aufweisen.

Versuche mit Luft und verschiedenen Flüssigkeiten haben ergeben, dass das Ventil in bestimmten erfindungsgemäßen Ausführungsformen im vorgesehenen Volumenstrombereich keine hörbaren Geräusche oder messbaren Druck-Schwingungen erzeugt. Bei Röntgenaufnahmen mit kontinuierlich durchströmender Luft konnte ein gleichmäßiger Öffnungsspalt beobachtet werden. Damit in Zusammenhang steht, dass die Ventilbauform in bestimmten erfindungsgemäßen Ausführungsformen eine besonders geringe Neigung zum Zusetzen durch anhaftende Partikel aufweist, insbesondere im Bezug auf anhaftendes gerinnendes Blut, da keine dichtbleibenden Abschnitte verbleiben, in denen die Blutbewegung zum Erliegen kommt. Die konische Bauform formt einen Strömungskanal welcher den vorgegebenen geometrischen Verlauf der Zuströmung von innen unten nach oben-außen am kontinuierlichsten vermittelt. Daraus resultieren auch ein geringerer Strömungsverlust der Gehäuseströmung (wegen weniger schroffer Strömungsumlenkung) und eine verbesserte Entlüftbarkeit des Haupt-Strömungsweges (wegen höherer mittlerer Strömungsgeschwindigkeit).

Bei sehr hohen Volumenströmen außerhalb des spezifizierten Bereichs wirkt die innere Gewölbedecke der Kappe in bestimmten erfindungsgemäßen Ausführungsformen als Bewegungsanschlag für den durch das Fluid angehobenen Haltering des Ventils. Der flache Bewegungsanschlag ist geometrisch so ausgeführt, dass keine Verklemmungsmöglichkeit des Ventils am Kappengewölbe entstehen kann. Bei den auftretenden typischen Sperrdrücken bis ca. 1,5 bar und im spezifizierten Volumenstrombereich von 0 bis 600 ml/min öffnet sich das Ventil alleine durch Biegebewegungen, wobei der innere Materialbereich des Ventils annähernd spielfrei zwischen Zentrierkonus und Anschlagstirn geführt wird und der Haltering in alle Raumrichtungen berührungsfrei von Kappe und Ventilsitz beabstandet ist. Durch Wegfall von Gleitvorgängen (etwa bei Rückschlagventilen mit verschieblichen Kugeln) ist die Kennlinie hoch reproduzierbar und annähernd hysteresefrei. Bei normalen Sperrdrücken bis ca. 2,5 bar legt sich der Dichtringbereich des Ventils kontinuierlich fester an den Dichtkonus des Ventilsitzes an und verstärkt die Dichtwirkung. Bei extremen Sperrdrücken bis ca. 5 bar beult sich der Federmembranbereich des Ventils nach unten aus und kehrt bei Druck-Zurücknahme in die korrekte Ausgangslage zurück. Bei normalen und extremen Sperrdrücken verhindert der Zentrieranschlagdom im Zentrum des Ventilsitzes eine axiale Verschiebung und eine axial-radiale Dejustierung der Ventilposition. Zusätzlich vermindert er den Strömungsraum und trägt damit zu gleichmäßiger Strömungsgeschwindigkeit bei.

Bei langen Phasen hoher Volumenströme relaxiert in bestimmten erfindungsgemäßen Ausführungsformen das Ventil um einige Millibar in Richtung sinkender Durchflusswiderstände, ein typisches Verhalten elastomerer Werkstoffe. Unter diesen Werkstoffen hat der Werkstoff Silikonkautschuk wegen besonders geringer Relaxation eine bedeutende Stellung. Da aber auch die Kappe durch die fortbestehende Rest-Axialkraft auf die Kappe in Richtung Ventilsitzgrund relaxiert, tritt eine kompensierende Wirkung ein, da diese Relaxier-Richtung mit der Richtung sich erhöhender Vorspannung übereinstimmt. Damit bietet die Kappen-Ventilbauform eine besonders geringe Relaxation der Druck-Volumenstrom-Kennlinie durch kompensierende Einbauanordnung. Durch geeignete Versuchsreihen kann die geometrische Gestaltung insbesondere des Kegelsitzbereiches der Kappe so optimiert werden, dass unter Berücksichtigung der Bauteiltoleranzen von Disposable und Maschine, der Behandlungstemperaturen und -zeiten und der Abnützungsvorgänge bei der Maschine eine optimale mittlere gegenseitige Kompensation der Relaxationen von Ventil und Kappe ermittelt und umgesetzt wird.

Die Dichtheit des abgerüsteten Kassettendisposables dort wo das Rückschlagventil vorgesehen ist, hat sich als so zuverlässig herausgestellt, dass eine Verschlusshülse (mit der entsprechenden Dichtfunktion speziell am automatischen Substituat-Konnektor beim Seriendisposable weggelassen werden kann, siehe hier das Bezugszeichen 41 in Fig. 1 der WO 2010/121819 A1. Damit einher geht eine weitere Vereinfachung des maschinenseitigen Aktor-Sensor-Kopplungs-Mechanismus, welcher nun keinen separaten Hub zur dichtenden Aktivierung der Verschlusshülse mehr ausführen muss und damit zugleich preisgünstiger und zuverlässiger arbeitet.

Die vorliegende Erfindung wird im Folgenden anhand der beigefügten Zeichnung, in welcher identische Bezugszeichen gleiche oder ähnliche Bauteile bezeichnen, exemplarisch erläutert. In den zum Teil stark vereinfachten Figuren gilt:
- **Fig. 1a bis 1c**: zeigen die Rückschlagventilanordnung in einer ersten erfindungsgemäßen beispielhaften Ausführungsform;
- **Fig. 1d**: zeigt eine Vergrößerung der Fig. 1c;
- **Fig. 2a bis 2d**: zeigen die einteilige Bauform einer weiteren erfindungsgemäßen Ausführungsform mit einer einteiligen krallenförmigen Rückschlagventilanordnung;
- **Fig. 3a bis 3c**: zeigen ein Ventil einer weiteren beispielhaften Ausführungsform mit einer Kappe aus Thermoplast;
- **Fig. 4a bis 4c**: zeigen eine Rückschlagventilanordnung in einer weiteren erfindungsgemäßen Ausführungsform der Funktionsvorrichtung;
- **Fig. 5a bis 5d**: zeigen eine weitere Bauform eines zweiteiligen vorgespannten Rückschlagventils mit Elastomer-Ventilkörper und Thermoplast-Rastkappe in einer weiteren erfindungsgemäßen Ausführungsform;
- **Fig. 6a bis 6d**: zeigen eine weitere erfindungsgemäße Ausführungsform der medizinischen Funktionsvorrichtung, hier rein exemplarisch eine Blutkassette, mit einer Rückschlagventilanordnung, in Explosionsdarstellung; und
- **Fig. 6e bis 6h**: zeigen die Ausführungsform der Fig. 6a bis 6d in zusammengefügtem Zustand in verschiedenen Ventilstellungen.

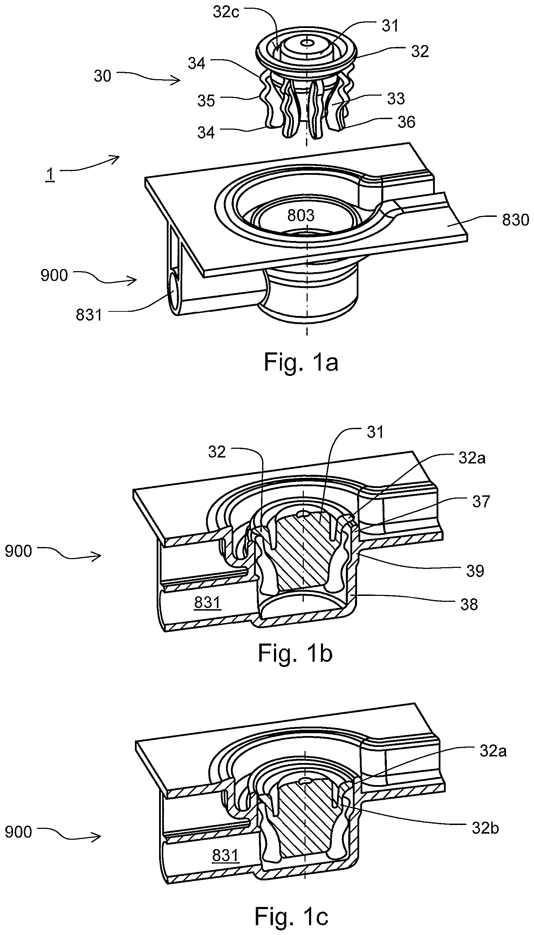

Die Fig. 1a bis 1c zeigen die Rückschlagventilanordnung 1 in einer ersten beispielhaften erfindungsgemäßen Ausführungsform der Funktionsvorrichtung 900.

Fig. 1a zeigt die Ventilanordnung 1 in Explosionsdarstellung, Fig. 1b in erster Stellung, d. h. in nicht aktivierter offener Initialstellung, und Fig. 1c in zweiter Stellung, d. h. "remanent" ( also "dauerhaft" oder "verbleibend") aktivierter Rückschlagventilstellung.

Ein einteiliger Ventilkörper 30 besteht vorzugsweise aus Silikonkautschuk. Ein mittig gelegener Zylinder oder Abschnitt des Ventilkörpers 30, hier als Kernzylinder 31 bezeichnet, dient mit seiner folienzugewandten Stirnfläche als hinreichend steifes Übertragungselement für den über die Kassettenfolie und den Aktivierungsaktor der Maschine (kurz auch Aktor, hier nicht dargestellt, siehe aber Fig. 6f) einzuleitenden axialen Aktivierungshub. Ein - beispielsweise kelchförmiger - Radialdichtsteg 32 ist umlaufend (d. h. in geschlossener Kontur oder mit geschlossenem Umfang) fluiddicht an den Kernzylinder 31 angebunden. Rippen, hier als Führungs-Klemm-Drainage-Rippen 33 bezeichnet, welche zum Führen, zum Klemmen und zum Drainieren dienen, sind vorzugsweise in gleichmäßiger und ungeradzahliger Teilung und in bevorzugter Anzahl von mehr als 2 mit einen äußeren Bereich des Kernzylinders 31 verbunden. Die Führungs-Klemm-Drainage-Rippen 33 halten den Ventilkörper 30 durch leichte radiale Verklemmung und unter Überbrückung der radialen Maßtoleranzen von Ventilkörper 30 und Ventilsitz 803 des Hartkörpers 830 der Kassette 900 zentrisch in einem Dichtzylinder 37 und einem Führungszylinder 38 (siehe Fig. 1b) des Ventilsitzes 803 der Kassette 900.

Dabei verhindern die Führungs-Klemm-Drainage-Rippen 33, beispielsweise mittels optionaler Klemm-Führungs-Noppen 34, welche auf ein und derselben Führungs-Klemm-Drainage-Rippe 33 axial auseinander liegen können, zugleich eine Verkippung des Ventilkörpers 30.

Benachbarte Führungs-Klemm-Drainage-Rippen 33 berühren einander im Beispiel der Fig. 1a bis 1d nicht.

Durch die Noppengeometrie, also durch die begrenzte axiale Erstreckung und die Balligkeit, kann eine toleranzenüberbrückende hohe Radialmaßüberlappung mit den benachbarten Zylindern 37 und 38, welche unterschiedlich große Durchmesser haben, wobei der Bereich des Durchmesserübergangs oder -sprungs als Raststufe 39 bezeichnet wird, bei definierter reibschlüssiger Zentrierung und Axialfixierung des Ventilkörpers 30 erreicht werden.

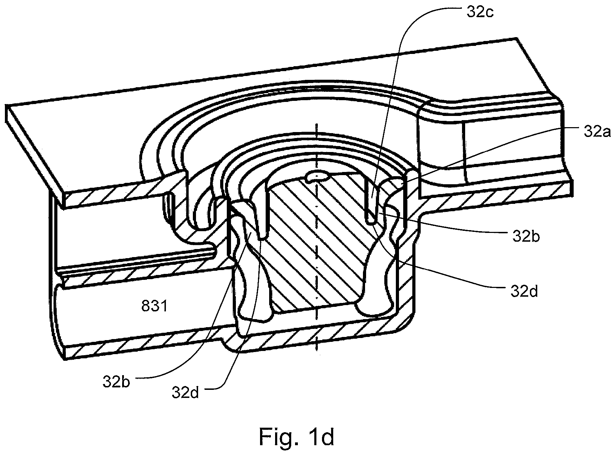

Das Bezugszeichen 32c bezeichnet einen umlaufenden Spalt, welcher mit Bezug zu Fig. 1d beschrieben ist.

Versuche haben gezeigt, dass die Slip-Stick-Effekte im Vergleich zu axial durchgängigen Rippen, d. h. Rippen ohne radial vorstehende Klemm-Führungs-Noppen 34, vorteilhaft geringer ausfallen und damit die Möglichkeit von Verkippungen beim Einbau und beim Start des Aktivierungshubes vorteilhaft verringert wird.

Fig. 1b zeigt die erste Stellung, die strömungsoffene Grundstellung, im Schnitt: Der Ventilkörper 30 ist sowohl durch Reibschluss der Klemm-Führungs-Noppen 34 als auch durch Anlage von Noppen, hier als Klemm-Rast-Noppen 35 bezeichnet, welche radial über die Klemm-Führungs-Noppen 34 hervorragen, an der Raststufe 39 in der strömungsoffenen Grundstellung (erste Stellung) fixiert. In dieser Stellung steht der Radialdichtsteg 32 axial außer Eingriff zum Dichtzylinder 37, und es besteht ein strömungsoffenes Ringvolumen (ein Sterilisierungsspalt). Erst durch Aufbringen einer Mindestaktivierungskraft (bevorzugt 20 bis 40 N) in axialer Richtung auf die Stirnfläche des Kernzylinders 31 setzt eine axiale Verschiebebewegung ein, indem die Summe der Haftreibekräfte der Klemm-Führungs-Noppen 34 und die resultierende radiale nach innen auf die Klemm-Rast-Noppen 35 wirkende Verformungskraft den eingestellten Grenzwert überschreiten. So wird der Schutz vor unbeabsichtigter Aktivierung während der Produktion der Kassette 900 gewährleistet.

Im Fußbereich des Ventilskörpers 30 - und nur vorzugsweise an einem unteren Ende dessen Führungs-Klemm-Drainage-Rippen 33 - vorgesehene Drainage-Fußrippen 36 begrenzen die Wirkung des Aktivierungshubs in axialer Richtung. Sie können ferner zusammen mit einigen oder allen anderen Rippen die gleichmäßige Entlüftbarkeit und Umströmbarkeit des Ventilkörpers sowie die sichere Sterilisierbarkeit mittels Gasen gewährleisten.

Fig. 1c zeigt die aktivierte Rückschlagventilstellung im Schnitt: Der dünnwandige Kelchmantel 32b und der etwas dickwandigere umlaufende Radialdichtsteg 32 mit spitz zulaufender Dichtkante 32a bilden zusammen mit dem Dichtzylinder 37 des Ventilsitzes 803 der Kassette 900 ein Rückschlag-Ventil-Dicht-System, nachdem der Aktivierungshub ausgeführt wurde. Dabei liegt die umlaufende Dichtkante 32a des elastomeren Ventilkörpers 30 unter radialer Vorspannung am Dichtzylinder 37 an und dichtet den folienseitigen Fluidraum gegen den konnektorseitigen Fluidraum, also gegen den Raum 831, aus welchem Fluid vom maschinenseitigen Konnektor auf das Ventil 30 zuströmen könnte.

Überschreitet der konnektorseitige Fluidraum einen definierten Öffnungsdruck (von beispielsweise 180 mbar) gegen den folienseitigen Fluidraum, dann steigt die radial nach innen wirkende Fluidkraft auf einen Kelchmantel 32b und es kommt zu zwei elastischen Verformungen, nämlich einer Durchmesserverminderung des Kelchmantels 32b und des Radialdichtstegs 32 und zu einem radialen Einknicken oder Falten des Radialdichtsteges 32. Auf diese Weise eröffnen sich an einer oder mehreren Stellen Strömungslumina zwischen der Dichtkante 32a und dem Dichtzylinder 37.

Herrscht im Fluidsystem der Kassette 900 folienseitig (und damit auf Seiten der Kanäle und Kammern) ein Unterdruck, welcher geringer als der Ansprechdruck ist, ein Gleichdruck oder ein Überdruck gegen die Konnektorseite, dann bleibt der folienseitige Fluidraum gegen den konnektorseitigen Fluidraum abgedichtet. Bei Überdruck wirkt die Druckbeaufschlagung radial nach außen auf den Kelchmantel 32b dichtpressungsverstärkend auf die Dichtkante 32a.

Die Fig. 1a bis 1c zeigen somit einen einteiligen Ventilkörper oder -einsatz des Rückschlagventils, gefertigt aus Elastomer, vom radialdichtenden Typ, in der erfindungsgemäßen Funktionsvorrichtung 900.

In weiteren, beispielhaften Ausführungsformen kann jedenfalls die in den Fig. 1a - 1c gezeigte Rückschlagventilanordnung 1 eine zusätzliche Stopfen-Komponente aufweisen. So kann der Kernzylinder 31 folienseitig oder konnektorseitig mit einem Sackloch versehen sein, welches durch einen mit Drainagerippen versehenen Stopfen aus Thermoplast (gleich oder ähnlich dem Thermoplastmaterial der Kassette) wieder aufgefüllt ist. Dieser Stopfen vermindert den Materialverbrauch beim elastomeren Ventilkörper und erhöht die axiale Steifigkeit des Kernzylinders 31. Auf diese Weise lässt sich der erforderliche Aktivierungs-Axialhub etwas verringern.

**Fig. 1d** zeigt eine Vergrößerung der Fig. 1c. Zu erkennen ist der Kelchmantel 32b im Schnitt.

Der Kelchmantel 32b kann als - beispielsweise konische oder zylindrische - Wandung verstanden werden, welcher sich - mehr oder weniger, oder zumindest abschnittsweise - in einer Auf-Zu-Richtung des Ventilkörpers 30 erstreckt.

Der Kelchmantel 32b kann als umlaufende Wandung verstanden werden, welcher wenigstens einen Abschnitt des Kernzylinders 31 umlaufend, also in geschlossener Form, umgibt. Dabei kann zwischen der Wandung des Kelchmantels 32c und der äußeren, seitlichen Begrenzung (Mantelfläche) des Kernzylinders 31 ein ebenfalls umlaufender Spalt 32c vorgesehen sein. Der Spalt kann einen Spaltgrund 32d haben, in welchem der Kernzylinder 31 oder zentrale Abschnitte hiervon in den Kelchmantel 32b übergehen.

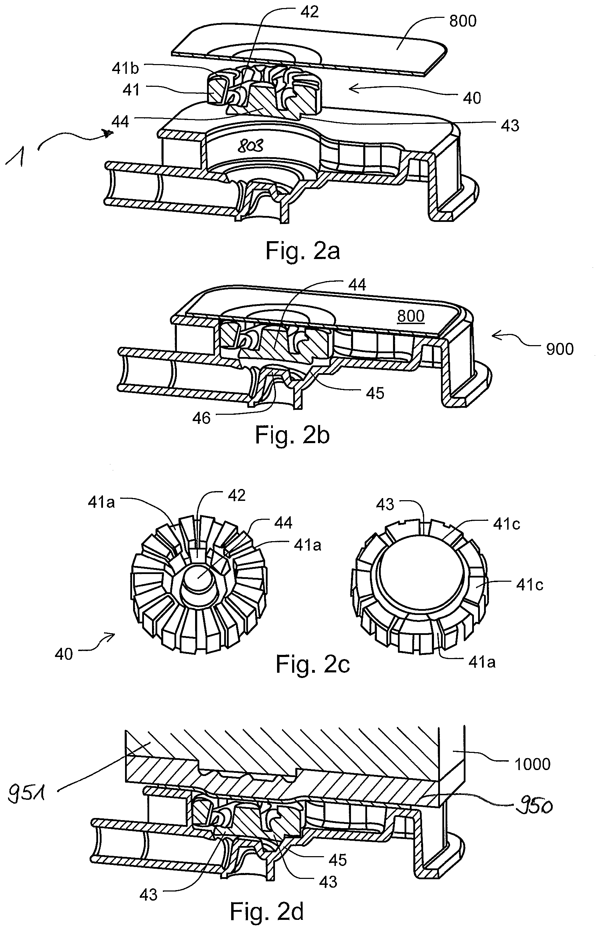

Die **Fig. 2a** bis **2d** zeigen die einteilige Bauform einer weiteren erfindungsgemäßen Ausführungsform, mit einer einteiligen krallenförmigen Rückschlagventilanordnung 1 in Explosionsdarstellung (siehe Fig. 2a), in einer ersten Stellung ("initial", Fig. 2b), in Perspektivansichten von oben und unten (Fig. 2c), sowie in einer zweiten Stellung ("aktiviert", Fig. 2d).

Ein Ventilkörper 40 besteht vorzugsweise aus einem elastomeren Werkstoff, vorzugsweise aus Silikonkautschuk. Er ist vorzugsweise so gestaltet, dass er in einem kostengünstigen Auf-Zu-Spritzgußwerkzeug hergestellt werden kann.

Ein Ringabschnitt, hier als Positionierungsring 41 bezeichnet, weist umlaufend Drainagestrukturen 41a auf, welche im eingebauten Zustand die Zugänglichkeit für Sterilisiergase gewährleisten. Eine - in Bezug auf die Darstellung der Figur 2a - obere Ringstirnfläche 41b des Positionierungsrings 41 weist in Richtung der Folie 800 der Kassette 900. In der ersten Stellung der Fig. 2b ist diese Fläche im Wesentlichen vollständig oder zumindest abschnittsweise vorzugsweise bündig mit der Folie 800 bzw. dem Kassettenrand, auf den die Folie 800 aufgelegt oder aufgeschweißt ist, wodurch sich eine einfache reibschlüssige Steckmontage mit axialem Bewegungsanschlag ergibt. Eine äußere Mantelfläche des Positionierungsrings 41 weist einen größeren Durchmesser auf als die zugeordnete Absatzbohrung in der Kassette 900. Über den Durchmesserunterschied, die Materialhärte und/oder die Gestaltung der Drainagestrukturen, welche Kontakt mit dem Innendurchmesser der Absatzbohrung haben können, ergibt sich die reibungsverursachte Haltekraft in der Kassette, welche das Ventil der Fig. 2a bis 2d in der zweiten Stellung hält. Sie wird beispielsweise auf einen Bereich von 20 bis 30 N eingestellt.

Die obere Ringstirnfläche 41b dient bei der Aktivierung in der Dialysemaschine 1000 der Einleitung der Aktivierungskraft (größer als beispielsweise 20 bis 30 N) bzw. des Aktivierungsweges von ca. 1,1 bis 1,4 mm. Durch die kompakte Gestaltung der Geometrie des Positionierungsrings 41 ergibt sich ein axial eher steifer Ringkörper, der den eingeleiteten Axialweg bei Montage und bei Aktivierung genauer "weiterleitet" als ein elastomerer Stössel, der zentral mittig angeordnet ist und einen kleinen Durchmesser aufweist wie beispielsweise die erfindungsgemäße Ausführungsform der Fig. 1. Somit wird etwas weniger Brutto-Aktivierungsweg bei Maschine 1000 und Folie 800 benötigt.

Eine untere Ringstirnfläche 41c dient als Bewegungsanschlag und damit der Kalibrierung des Öffnungsdrucks und der Durchlasskennlinie in der zweiten Stellung ("aktiviert").

Ein Abschnitt, der hier als Durchlassringzone 42 bezeichnet wird, besteht aus einzelnen, schleifenförmig gewundenen Einzelstegen mit zwischen diesen angeordneten Spalten oder weist solche auf. Die Durchlassringzone 42 dient dem Fluiddurchlass in der Stellung "aktiviert", hält den Ventilkern in Sollstellung und bildet eine axial federnde und Schiefstellungen ausgleichende Haltestruktur.

Der exemplarisch pilzförmige Ventilkern weist einen steiferen Kernbereich 44 auf, der in beide axiale Bewegungsrichtungen als Bewegungsbegrenzung gegen die Folie 800 und gegen einen kassettenseitigen Stössel 46 dient. Diese Anschläge werden nur berührt, wenn der festgelegte Druck- und Volumenstrombereich verlassen wird.

Eine Dichtringzone 43 des Ventils 40, welche hier exemplarisch als umlaufende Lippe oder umlaufender Rand ausgestaltet ist, weist in der ersten Stellung einen Ringspalt von rein beispielhaft ca. 0,4 mm zu einem konischen Dichtsitz 45 des Körpers der Kassette 900 auf, was dazu beiträgt, dass eine Gassterilisation zuverlässig möglich ist. In der zweiten Stellung dagegen dichtet die Dichtringzone 43 - beispielsweise unter Mindest-Vorspannung von ca. 0,4 mm - gegen einen konischen Dichtsitz 45 des Körpers der Kassette 900 ab. Man kann die Dichtungsart mit nur geringen geometrischen Abwandlungen auch als axial dichtend ausführen, was ebenfalls von der vorliegenden Erfindung umfasst ist.

Die Fig. 2a bis 2d zeigen somit einen einteiligen Ventilkörper oder -einsatz aus Elastomer, welcher konisch dichtet.

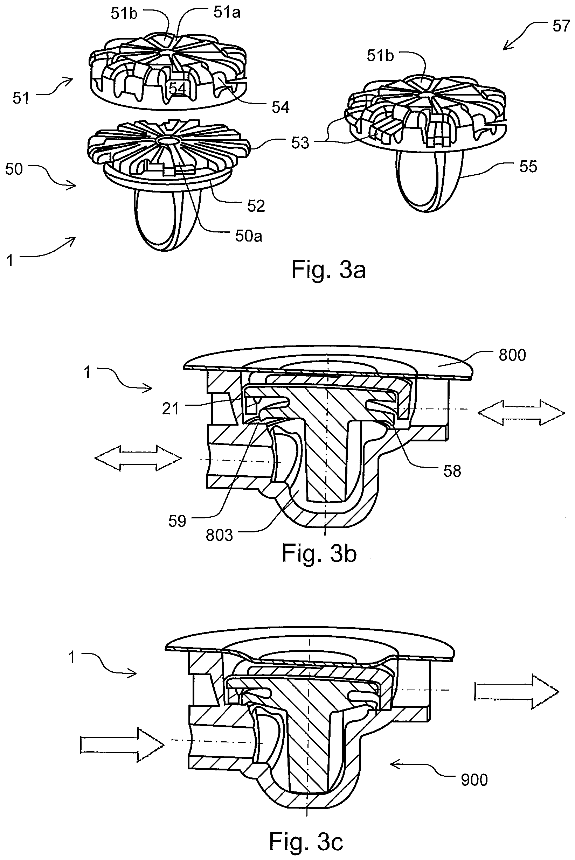

Die **Fig. 3a** bis **3c** zeigen eine weitere beispielhafte Ausführungsform eines Ventils mit einer Kappe 51, vorzugsweise aus Thermoplast (vorzugsweise PP). Fig. 3a zeigt einen Ventilkörper 50 und die Kappe 51 des Ventils in Explosionsdarstellung (links) und im zusammengebauten Zustand (rechts). Fig. 3b zeigt das Ventil in der ersten Stellung ("initial"), Fig. 3c in der zweiten Stellung ("aktiviert").

Der Ventilkörper 50 bildet zusammen mit der Kappe 51 den Aktivierungs- und Haltering des Rückschlagventils bzw. der Rückschlagventil-Anordnung. Nach der Vormontage des Ventilkörpers 50, welcher einen Dichtring 52 aufweist, in der Kappe 51 ragen Noppen 53 des Ventilkörpers 50 radial aus Durchbrüchen 54 der Kappe 51 nach außen. Sowohl Ventilkörper 50 als auch Kappe 51 weisen optional Drainagestrukturen 50a und 51a zur optimalen Gas- und/oder Dampf-Sterilisierbarkeit auf. Sie sind geometrisch so aufeinander abgestimmt, dass der Ventilkörper 50 axial mit seiner Aktivierungs-Stirnfläche unter leichter Vorspannung an der innenseitigen Aktivierungs-Stirnfläche der Kappe 51 anliegt.

Die Noppen 53 der Kappe 51 oder des Halterings bilden zusammen mit den Rippen 55 des Ventilfußes, in welchen sich in Fig. 3a der Dichtring 52 beispielhaft fortsetzt oder an welchen sich der Dichtring 52 anschließt, eine spielfrei zentrierende und radial vorgespannte Halterung der Ventilbaugruppe im Ventilsitz 803 der Kassette 900, welche ein eigenständiges Übergehen des Ventils aus der aktivierten Stellung in die Initialstellung verhindert. Die vorstehend genannten Komponenten können gemeinsam als Haltekopf 57 bezeichnet werden.

Durch das Zusammenwirken mit dem als Durchmesserabsatz 21 im Halteringbereich der Kassette 900 bezeichneten Stufe innerhalb des zylinderförmigen Abschnitts des Ventilsitzes 803 der Kassette 900 beträgt die erforderliche Verschiebe-Axialkraft im Ventilsitz 803 beispielsweise ca. 20 N in Ausbaurichtung und 30 N in Aktivierungsrichtung. Dies reicht einerseits für eine ausreichend sichere Fixierung in der gasoffenen ersten Stellung während der Produktion und andererseits für eine geringe erforderliche Aktivierungskraft in der Behandlungsmaschine.

Durchbrüche 54 und Drainagestrukturen 51a dienen - vorzugsweise in Verbindung mit der Balligkeit der äußeren Aktivierungsstirnfläche 51b der Kappe 51 - auch der Entlüftungsmöglichkeit und dem Durchfluss der Behandlungsflüssigkeiten durch die Ventilanordnung 1.

Die im Vergleich zum Ventilkörper 50 sehr steife, jedenfalls aber steifere Kappe 51 erlaubt es, die Ventilbaugruppe axial auf etwa +/- 0,2 mm genau in der ersten Stellung einzubauen. Zur Überwindung des Sterilisierungs-Spaltes von mindestens 0,2 mm und zum Aufbau einer ausreichenden Ventilvorspannung ergibt sich ein Nenn-Aktivierungsweg von ca. 1 mm. Da nur relativ geringe Axialkräfte zum Verschieben der reibschlüssig gehaltenen Ventilbaugruppe erforderlich sind, benötigt die Aktor-Sensor-Baugruppe der Maschine 1000 etwa einen Vorstand der Aktivierungsnase von ca. 1,2 bis 1,4 mm zur sicheren Aktivierung, also der zweiten Stellung. Diese ist gegeben bzw. erreicht, wenn sich eine Ringstirnfläche 58 des Ventilkörpers 50 auf eine Ringstirnfläche 59 des Ventilsitzes 803 der Kassette 900 bis zur flächigen Berührung angenähert hat. Selbst bei einer maximalen Aktivierungskraft von beispielsweise 60 N von der Maschine auf die Kappe 51 verbeult sich diese axial nur um etwa 0,05 mm.

Die in den Fig. 3a bis 3c angegebenen Pfeile oder Doppelpfeile zeigen an, in welchen Richtungen die Ventilanordnung jeweils durchströmbar sind: In Fig. 3b sind dies aufgrund der offenen, ersten Stellung beide Durchströmrichtungen, also sowohl von links nach rechts als auch umgekehrt. In Fig. 3c, welche die aktivierte, zweite Stellung zeigt, in welcher die Ventilanordnung als Rückschlagventil wirkt, ist dies nicht der Fall - hier kann die Ventilanordnung, ausreichenden Druck vorausgesetzt, nur von links nach rechts durchströmt werden.

Die Fig. 3a bis 3c zeigen somit eine zweiteilige Ventilanordnung oder einen zweiteiligen Ventileinsatz aus Elastomer oder Thermoplast, welcher flach dichtet, bei entsprechender Anpassung allerdings auch konisch dichten könnte.

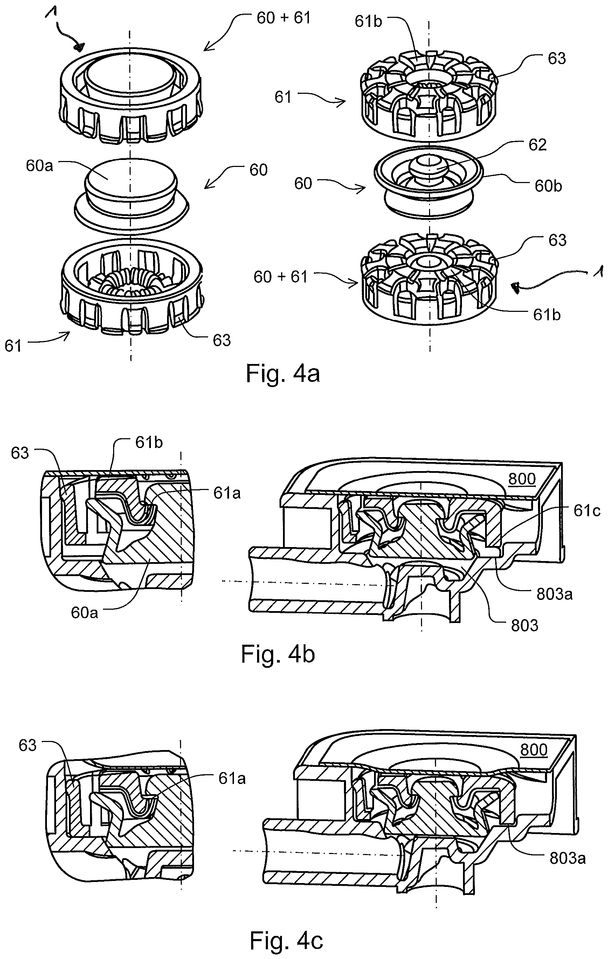

Die **Fig. 4a** bis **4c** zeigen eine Rückschlagventilanordnung 1 einer weiteren, beispielhaften erfindungsgemäßen Ausführungsform. Es handelt sich um eine zweiteilige, konisch dichtende Ventilbaugruppe mit einer Ventilkomponente aus Elastomer (vorzugsweise Silikonkautschuk) und einer Kappenkomponente aus Thermoplast (vorzugsweise PP), welche in einen Ventilsitz 803 eingesetzt werden. Dabei zeigt die Fig. 4a die Ventilbaugruppe in einer Explosionsdarstellung von unten (links) und von oben betrachtet (rechts), wobei die oben bzw. unten dargestellte und die jeweils in der Mitte dargestellte Komponente unten bzw. oben (bezogen auf das Zeichnungsblatt) noch einmal zusammengefügt gezeigt sind. Die Fig. 4b zeigt die Ventilanordnung 1 in sog. "vorgespannter" (Begriff wird unten erläutert) erster Stellung ("initial"), und die Fig. 4c zeigt die Ventilanordnung 1 in zweiter Stellung ("aktiviert"). Die jeweils links wiedergegeben Darstellungen der Fig. 4b und 4c zeigen zur jeweils rechten Darstellung zugehörige Ausschnittsvergrößerungen.

Der Ventilkörper 60 hat hier exemplarisch die Form eines Bechers oder Napfes mit faltenbalgartigem Mantel, weitgehend flachem, vergleichsweise steifem Ventilboden 60a und mit einem zentral und steif an den Ventilboden 60a angebundenen Zuganker 62. Der Ventilkörper 60 wird mittels des Zugankers 62 bei der Vormontage in eine passende, zentrale Rastöffnung der Kappe 61 eingerastet.

Ungeachtet der in Fig. 4a bis 4c gezeigten Ausgestaltung des Ventilkörpers 60 kann dieser die Gestalt eines Gefäßes haben, welches an einer Stirnseite hiervon offen ist, wobei sich im Inneren des Ventilkörpers 60 ein Zuganker zur offenen Stirnseite hin erstreckt und ggf. aus dieser herausragt.

Die dem Ventilboden 60a abgewandte Ringstirn 60b des Faltenbalgmantels sitzt radial unter leichter Vorspannung von beispielsweise ca. 0,1 mm und axial unter vorzugsweise größerer Vorspannung von beispielsweise ca. 0,5 mm in der Kappe 61. Dabei wird der Faltenbalg entsprechend komprimiert, und die axiale Vorspannung wird über den eingerasteten relativ steifen Zuganker 62 aufrechterhalten. Das Ventil ist also vorgespannt vollständig spielfrei radial zentriert und behält diese Eigenschaft auch bei späterer Aktivierung der Kappe 61 und bei Öffnung des Ventils mittels durchströmender Fluide bei. Diese axiale Vorspannung kann hierin als Vorspannung des Rückschlagventils bezeichnet werden. Diese Eigenschaft wird erst möglich durch den Einsatz der Kappe 61 und bringt Vorteile mit sich. Denn ohne Vorspannung müsste der Aktivierungsweg so groß gewählt werden, dass er im vorliegenden Beispiel in der ersten Stellung einen Sterilisationsspalt von ca. 0,2 mm gewährleistet, und dies unter Berücksichtigung aller Toleranzen der Disposable-Bauteile einschließlich der Disposable-Fertigung. Ferner müsste der Aktivierungsweg so groß gewählt sein, dass er in der ersten Stellung unter Berücksichtigung aller Toleranzen aller Bauteile einschließlich der Maschine eine axiale Vorspannung des Dichtrings der Elastomerkomponente gegen den (hier konischen) Dichtring des Ventilsitzes aufweist, die zur erwünschten sicheren Fluiddichtheit bis zum gewünschten Öffnungsdruck führt. Bei einem gewünschten Öffnungsdruck von beispielsweise 180 mbar wird beispielweise eine axiale Vorspannkraft von ca. 1 N benötigt. Beträgt beispielweise der sicher realisierbare Aktivierungsweg der Kappe 0,8 mm (und der damit einhergehende auf der Maschinenseite vorgehaltene Brutto-Aktivierungsweg ca. 1,4 mm) und betragen die Toleranzen des vormontierten Disposables zusammen 0,2 mm, so bleiben für die eigentliche axiale Vorspannung des Ventils in der zweiten Stellung noch 0,4 mm. Die mittlere Federrate der axialen Nachgiebigkeit des Rückschlagventils beträgt also 2,5 N/mm bzw. 45 mbar/0,1 mm. Bei einer Unsicherheit der Abmessungen von 0,2 mm führt dies zu einer Unsicherheit des Öffnungsdrucks von 90 mbar, also von 50 % des Sollwertes. Bei Steigerung des Fluiddurchsatzes auf beispielweise 600 ml/min würde dies beispielweise einen Druckverlust von 600 mbar ergeben.

Mittels der vorstehend beschriebenen Vorspannung und eines Verzugs von beispielsweise 0,4 mm erreicht man bei den gleichen Weg- und Toleranzverhältnissen wie zuvor genannt auf vorteilhafte Weise, dass der Zuganker 62 in Stellung 'aktiviert' des Ventils gerade noch sicher von einer dem Zuganker 62 zugeordneten Ringfläche 61a der Kappe 61 axial abhebt. Für die eigentliche axiale Vorspannung des Ventils ergeben sich nun 0,4 mm + 0,4 mm = 0,8 mm. Die mittlere Federrate des Rückschlagventils 1 muss also nun nur 1,25 N/mm bzw. 22,5 mbar/0,1 mm betragen, damit der gewünschte Öffnungsdruck wiederum 180 mbar beträgt, diesmal aber mit der halbierten Unsicherheit von 45 mbar, also von nur mehr 25 % des Sollwertes. Durch die flacher gewordene Feder-Kennlinie erhält man auch eine flachere Druckverlust-Volumenstrom-Kennlinie, so dass man beispielsweise bei einem Durchsatz von 600 ml/min einen Druckverlust von ca. 450 mbar erhält. Dieser geringere Druckverlust wirkt sich wiederum positiv auf die Genauigkeit der Kennlinie aus und hat zusätzlich weitere positive Eigenschaften: Die Pumpen in der Kassette 900 und in der Maschine 1000 können beispielsweise bei der Anwendung des hier beschriebenen Rückschlagventils als Einlass-Rückschlagventil der Dialysierflüssigkeit entsprechend druckschwächer ausgelegt werden. Das Einlass-Rückschlagventil für die venöse Luer-Zugabe kann wegen der größeren Reproduzierbarkeit des Öffnungsdrucks auf einen kleineren Öffnungsdruck hin ausgelegt werden, wodurch die Bedienperson weniger Kraft benötigt, um mittels einer Spritze deren Medikamenteninhalt in den Blutkreislauf hineinzudrücken. Da dieses Rückschlagventil auch für die arterielle Blutrückgabe bei Behandlungsende genutzt werden kann, sinken mit den Druckverlusten auch die Strömungsgeschwindigkeiten und Scherraten, wodurch die Hämolyse entsprechend vermindert wird.

Die Kappe 61, optional aus dem relativ steifen Werkstoff PP (Biege-E-Modul ca. 1750 N/mm), hat wiederum die Aufgabe, den vorzugsweise elastomeren Ventilkörper 60, vorzugsweise aus dem relativ nachgiebigen Werkstoff Silikonkautschuk (Biege-E-Modul ca. 15 N/mm), spielfrei vorgespannt in sich aufzunehmen, mit geringer axialer Toleranz von beispielsweise +/- 0,1 mm in der ersten Stellung zu halten und mit ebenso geringer Toleranz in die zweite Stellung zu überführen, in welcher sich eine folienabgewandte Ringfläche 61c der Kappe 61 und eine Ringfläche 803a des Ventilsitzes 803 berühren. Dabei wird wiederum die deutlich größere Toleranz des von der Maschine 1000 über die optionale Aktor-Sensor-Matte 950 oder direkt mittels Aktors 951 (siehe Fig. 6f) und die Folie 800 übertragenen Aktivierweges und zugehöriger Aktivierungs-Axialkraft vom Rückschlagventil ferngehalten und entkoppelt, was ohne zwischen Folie 800 und Ventilkörper 60 angeordneter steifer Kappe 61 nicht erreicht werden kann. Auch in der hier dargestellten Ausführungsform der Kappe 61 wird diese auf einfache Weise mit ihrer folienzugewandten Ringstirnfläche 61b bündig mit der Folienebene der Kanalränder der Kassette 900 eingebaut. Die Kappe 61 weist sich radial erstreckende, radial vorstehende und/oder radial ausfedernde Zungen 63 auf. Sie sorgen für den definierten und sicheren reibschlüssigen Halt der Ventilbaugruppe in der ersten Stellung (dabei könnte die vorliegende Ventilbauform erfindungsgemäß optional aber als Variante auch so ausgeführt sein, dass radial von der Faltenbalg-Ringstirnfläche wieder Zungen 63 abstehen, die durch Durchlässe in der Kappe 61 hindurchgreifen und so reibschlüssig für den sicheren Halt in beiden Ventilstellungen sorgen). Die Federzungen-Funktionalität wird ausführlicher mit Bezug auf die Fig. 6a bis 6d beschrieben. Die Kappe 61 ist optional wiederum auf vorzugsweise allen Seiten mit zahlreichen Drainage- und Durchbruch-Strukturen ausgestattet, welche die sichere Gassterilisation und Entlüftung gewährleisten und den druckverlustarmen Durchtritt der Behandlungs-Fluide in Öffnungsrichtung sicherstellen (Druckverlustanteil ohne Ventil kleiner als ca. 10 mbar bei 600 ml/min).

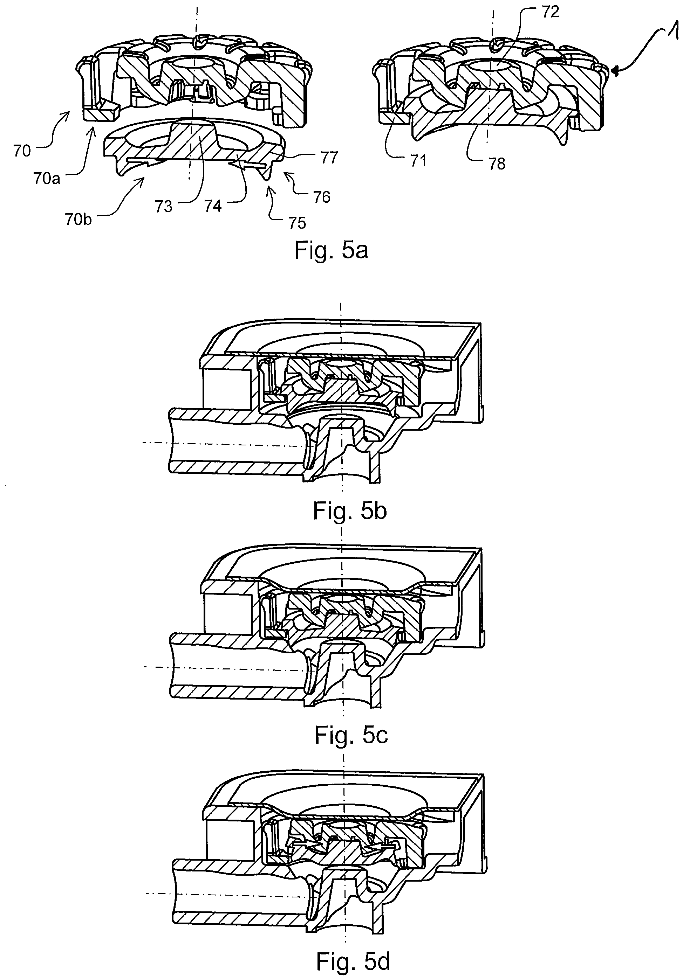

Die **Fig. 5a** bis **5d** zeigen eine weitere Bauform eines zweiteiligen vorgespannten Rückschlagventils mit Elastomer-Ventilkörper und Thermoplast-Rastkappe, welche in einen Ventilsitz der erfindungsgemäßen Funktionsvorrichtung eingesetzt werden. Die Fig. 5a zeigt die Ventilbaugruppe in Explosionsdarstellung (links) und zusammengebaut (rechts). Die Fig. 5b zeigt die Ventilanordnung in vorgespannter, erster Stellung ("initial"), die Fig. 5c zeigt sie in der zweiten Stellung ("aktiviert"), jedoch ohne Fluss, und auch die Fig. 5d zeigt sie in der zweiten Stellung, jedoch bei maximalem Fluss.

Die Rastkappe 70 behält weitgehend ihre aus den vorangegangenen Figuren bekannte Bauform, weist nun aber zusätzlich an der unteren Ringstirn 70a mehrere radial nach innen kragende Haltevorsprünge oder Haltenasen 71 auf. Die exemplarische Rastkappe 70 der Fig. 5a bis 5d ist so konzipiert, dass sie in einem Vielfach-Auf-Zu-Spritzgießwerkzeug mit optionalen, jeweils zentralen Heißkanal-Anspritzungen und äußerst günstig hergestellt werden kann.

Die Rastkappe 70 weist eine zentrale Aktor-Angriffsfläche 72 auf, auf welche ein Aktor 951 durch die Folie 800 hindurch auf die Aktor-Angriffsfläche 72 einwirken kann.

Der Ventilkörper 70b weist einen zentralen konischen Dorn 73 auf, welcher spielfrei in dem entsprechenden Konus der Kappe 70 aufgenommen wird. Eine Verbindungsmembran 74 erstreckt sich zwischen Dorn 73 und einem Halte-Dicht-Ring 75-76-77.

Der Ventilkörper 70b weist im nicht eingebauten Zustand eine leichte Verwölbung einer mittig angeordneten Verbindungsmembran oder Membran 78 nach unten (bezogen auf die Darstellung in Fig. 5a), oder der Folie abgewandt, auf. Bei der Vormontage des Ventilkörpers 70b in der Kappe 70 durch Einrasten eines Auflageflansches 76 des Halte-Dicht-Rings 75-76-77 bis zu dessen Verzahnung mit den Haltenasen 71 der Kappe 70 derart, dass der Auflageflansch 76 auf den Haltenasen 71 zum Liegen kommt, wird diese Wölbung bereits neutralisiert bzw. die Membran 78 elastisch bereits so umgeformt, dass eine leichte Wölbung in die Gegenrichtung, also nach oben, siehe Fig. 5b, zustande kommt. Die Wölbung in Gegenrichtung verstärkt sich bei der Aktivierung, also beim Übergang in die zweite Stellung, siehe Fig. 5c, und ein weiteres Mal bei Durchströmung mit Behandlungsfluiden, siehe Fig. 5d.

Der Halte-Dicht-Ring 75-76-77 ist im Verhältnis zur Membran 78 kompakt und/oder dickwandig ausgeführt. Bei der axialen Verlagerung des Halte-Dicht-Rings 75-76-77 relativ zum Dorn 73 entsteht einerseits eine Biegespannung in der Membran 78, die beständig dahingehend wirkt, den Halte-Dicht-Ring 75-76-77 wieder nach unten in die spritzgießtechnisch vorgegebene Ursprungsgestalt zu verformen. Andererseits entsteht bei der axialen Verschiebung des Halte-Dicht-Rings 75-76-77 auch eine Ringzugspannung im Halte-Dicht-Ring 75-76-77, die bestrebt ist, den Halte-Dicht-Ring 75-76-77 zu seinem initialen Durchmesser zurückzuverformen. Den größten Durchmesser hat der Halte-Dicht-Ring 75-76-77 dabei in der Situation, bei der die Membran 78 im wesentlichen flach verformt ist und deshalb eine Druckspannung radial nach außen auf den Halte-Dicht-Ring 75-76-77 ausübt. Sobald dieser neutrale Punkt überschritten ist, sinkt diese Druckspannung wieder und der Halte-Dicht-Ring 75-76-77 trägt eine axiale Kraftkomponente bei, die ihn in die entgegengesetzte Lage (in Ventilöffnungsrichtung) zu verschieben versucht. Diese axiale Kraftkomponente überlagert sich mit der beständig zunehmenden Biegespannung in der Membran 78 und führt zu einer Verminderung der Federrate des Ventilöffungsverhaltens. Erst durch die Vorspannung in der Rastkappe 70 gelangt man bei der eigentlichen Vorspannung des Ventilkörpers 70b in der zweiten Stellung zu ausreichend hohen Wegen, bei denen sich dann dieser Umwölbungs-Effekt zur weiteren Kennlinien-Verflachung ausnutzen lässt.

Der Halte-Dicht-Ring 75-76-77 kann ein umlaufender, radialer Abschnitt sein, welcher zwei von einander verschiedene Durchmesser mit einer zwischen beiden liegenden Stufe aufweist, und welcher den Ventilkörper in einen Abschnitt hiervon radial abschließt (also der radiale Rand ist).

Die Fig. 5a bis 5c zeigen damit eine Rückschlagventilanordnung in Gestalt eines Dornteller-in-Rastkappe oder eines in einen Konus der Rastkappe eingeführten Dorns des Ventilkörpers, welcher zweiteilig ist, aus Elastomer/Thermoplast hergestellt ist und konisch dichtet.

Die **Fig. 6a** bis **6d** zeigen eine weitere erfindungsgemäße Ausführungsform der medizinischen Funktionsvorrichtung, hier rein exemplarisch eine Blutkassette 900 mit einer Rückschlagventilanordnung 1. Sie zeigen in Zusammenschau die Bestandteile der Rückschlagventilanordnung 1 in perspektivischer Explosionsdarstellung im Halbschnitt. Die **Kassettenbaugruppe** besteht aus einer halboffenen Behandlungskassette 900 bzw. dessen nach oben offenen Ventilsitz 803, vorzugsweise aus PP oder anderen thermoplastischen Spritzgießwerkstoffen wie z. B. Polyvinylchlorid (kurz: PVC) oder Polycarbonat (kurz: PC), und einer kanalrandbündig aufgebrachten, die Kassette 900 verschließende aber ausreichend dehnfähige Abdeckfolie 800, siehe Fig. 6a, vorzugsweise aus PP-TPE-Mehrschichtextrudaten (mit "TPE" für thermoplastische Elastomere) oder anderen biegeweichen Extrusionswerkstoffen wie z. B. Weich-PVC oder thermoplastisches Polyurethan (kurz: TPU).

Die **Folie** 800 hat eine Stärke von beispielsweise 0,24 mm und ist randbündig auf den umlaufenden **Foliensteg** 814 aufgeschweißt, aufgeklebt oder aufgepresst. Die Folie ist im Initialzustand, d. h. in der ersten Stellung, bevorzugt eben angeordnet, damit sie möglichst auf der Kassette 900 angebracht werden kann; sie kann aber auch vorteilhaft für eine besonders geringe oder nicht auftretende Dehnbeanspruchung der Folie 800 nach oben (d. h. weg von der Kassette 900) vorgebeult sein, so dass bei der zur Aktivierung erforderlichen Verschiebung oder Bewegung lediglich eine Umbeulung stattfindet. Bei der ebenen Folienanordnung ergibt sich durch den geringen Aktivierungshub von beispielsweise 0,8 mm eine Foliendehnung von weniger als 2%. Der verwendete preisgünstige Folientyp mit geringen Elastomeranteilen kann diese Dehnung ohne Zerstörungsgefahr aufnehmen, wobei die Folie 800 bei der Ventil-Aktivierung durch die ebengenannte Dehnung zunächst eine Kraft von ca. maximal 20 N aufnimmt, welche anfänglich zusätzlich aufzubringen ist, sich im Laufe der Behandlung durch plastische Umformung der Folie auf annähernd Null abbaut.

Der **Ventilsitz** 803 der Fig. 6d ist exemplarisch geometrisch so ausgelegt, dass er als Spritzgießteil zur Entformung keine Hinterschnitte aufweist. Da sich die Rückschlagventilfunktion in der Behandlungskassette 900 immer auf Fluide bezieht, die von Schlauchleitungen kommen und in die Kassette 900 einströmen sollen, benötigt die Kassette 900 ohnehin an den betroffenen Stellen eine zylindrische **Rohranordnung** 804, die über ein Loch in die Kanal- und Kammeranordnung der Kassette 900 (also hier im unteren Bereich des Ventilsitzes 803) mündet. Insofern entstehen für das Integrieren von Rückschlag-Ventilsitzen der dargestellten Bauform in Kassetten 900 annähernd keine Mehrkosten.

Der Ventilsitz 803 enthält wenigstens einen abgehenden Fluidkanal 808.

Der Ventilsitz 803 ist in Einspann- bzw. Aktivierungsrichtung besonders steif ausgestaltet durch zylindrische, annähernd vertikale Zylinderwände 836 und durch eine großflächige Ventilsitz-Stützstirn 837, welche eine umlaufende Fläche sein kann, und welche senkrecht oder im Wesentlichen senkrecht zu den Zylinderwänden 836 angeordnet sein und/oder in diese übergehen kann.

Die - nur vorzugsweise zylindrische - **Rohranordnung** 804 kann in die Zylinderwände 836 münden.

Der abgehende Fluidkanal 808 geht vorzugsweise oberhalb der Zylinderwände 836 ab.

Die Ventilbaugruppe besteht aus der Kappe 801 (vorzugsweise aus PP oder aus einem anderen relativ steifen thermoplastischen Spritzgießwerkstoff wie PC oder Hart-PVC), siehe Fig. 6b, und dem Ventilkörper 802, siehe Fig. 6c (vorzugsweise aus LSR (Liquid Silicon Rubber), einem preisgünstigen Massen-Elastomer-Kunststoff mit ausreichend geringem Druckverformungsrest, guter Sterilisationsbeständigkeit und guter Hämokompatibilität).

Die **Kappe 801** hat die Form eines Gewölbes mit mehreren radial nach außen und axial nach oben offenen **Durchbrüchen** 805. In diesen Durchbrüchen 805 sind **Rastzungen** 806 angeordnet, die sich beispielsweise mit geringer Kraft von ca. 1 N pro 0,2 mm Radialauslenkung der Spitzen der Rastzungen 806 radial nach innen biegen lassen. Die Rastzungen 806 verschließen die Durchbrüche 805 nur teilweise, beispielsweise zu ca. 30%.

Bei der bevorzugten Ausführungsform ist die Anzahl der Durchbrüche 805 und der Rastzungen 806 bevorzugt jeweils eine ungerade, da ungerade Teilungen die Zentriergenauigkeit der Kappe 801 im Ventilsitz 803 erhöhen (einer Rastzunge 806 stehen in dieser beispielhaften Ausführungsform immer zwei zweidimensional zentrierend wirkende Durchbrüche 805 gegenüber bzw. ist diesen jeweils unter Beibehaltung eines Spalts benachbart). Die Anzahl der Durchbrüche bzw. Rastzungen beträgt bevorzugt 9 und kann bei einem Ventilsitzaußendurchmesser von 13 mm vorteilhaft in einem Bereich von 7 bis 11 liegen.

Durch die relativ große Anzahl von Durchbrüchen 805 bzw. Rastzungen 806 wird es möglich, die Ventilbaugruppe ohne festgelegte Drehorientierung in den Ventilsitz 803 einzubauen. Auch wenn - vorzugsweise senkrechte oder im Wesentlichen senkrechte - Zylinderwände, hier als Kappenaufnahme-Ventilsitz-Zylinderwände 807 und 817 bezeichnet, an ein- bis drei Stellen durch abgehende Fluidkanäle unterbrochen sind, reichen die verbleibenden Wandsegmente dieser Zylinderwände bzw. des Ventilsitzes 803 aus, um die Kappe 801 ausreichend genau zu zentrieren, in den gewünschten Stellungen zu halten und durch die Durchbrüche 805 hindurch einen hinreichend geringen Strömungswiderstand für die Behandlungsfluide zu erzielen.

Bei maximalem Fluss von etwa 600 ml/min liegen die reinen Durchflusswiderstände der Kappe 801 im eingebauten und aktivierten Zustand beispielsweise bei ca. 20 mbar und weisen demnach nur ca. 4 bis 5 % des Gesamtströmungswiderstandes mit eingebautem durchströmtem Rückschlagventil 1 auf.

Die Kappe 801 ist vorzugsweise so konstruiert, dass sie in schieberlosen Auf-Zu-Spritzgießwerkzeugen mit hohen Kavitätenzahlen besonders preisgünstig hergestellt werden kann. Sie bildet die bisher kostengünstigste gefundene Bauform des remanent aktivierbaren Rückschlagventils einer erfindungsgemäßen Funktionsvorrichtung 900.

Die Kappe 801 weist eine umlaufende, vorzugsweise scharfe Kante, bezeichnet mit den Bezugszeichen 809 und 827, auf, die sich bei - vorzugsweise nach unten offenen, beispielsweise U-förmigen - Stützbögen 810, welcher der Kappe 801 Stabilität verleihen, und den biegsamen Rastzungen 806 auf gleicher Höhe befinden und dabei die Haupttrennebene des Spritzgußwerkzeugs bilden. Aus der Not, dass es bei Spritzgießbauteilen (vor allem wenn diese mittels Vielkavitäten-Werkzeugen hergestellt sind) in der Haupttrennebene immer zu Graten und immer zu Formversatz kommt, wird hier eine Tugend gemacht, indem die scharfe Gratkante und ein in die Konstruktion einbezogener systematischer radialer Formversatz in Gestalt der Kante als Funktionselemente zur Ventilfunktion bewusst eingeplant sind (Erklärung weiter unten).

Eine obere Fläche oder Ebene, hier als obere Kappen-Ringstirn 811 bezeichnet, bildet die höchste Partie der Gewölbekonstruktion, zu welcher die Stützbögen 810 zählen, und stellt durch die Folie 800 hindurch die mechanische Schnittstelle zur Einleitung von Aktivierungskraft, Aktivierungsweg und Haltekraft durch die Aktor-Sensor-Einheit (kurz: ASE) der Maschine 1000 dar. Sie stellt eine durch vorzugsweise radial verlaufende Strukturierungsrillen 816 unterbrochene, vorzugsweise ebene Ringstirnfläche dar, deren Durchmesser in einem Bereich von 6,5 bis 8,5 mm liegen kann. Damit liegen der Durchmesser und die Größe der Ringstirnfläche, als der wirksamen Kontaktfläche für einen Aktor 951 (siehe Fig. 6f) während der Aktivierung, in einem Bereich, der günstig dafür ist, dem Aktivierungshöcker (nicht dargestellt) der Aktor-Sensor-Einheit genügend Übertragungsfläche für Kraft und Weg zu bieten, gleichzeitig aber weit genug vom maximalen Sitzdurchmesser von 13 mm entfernt zu sein, so dass einerseits die Dehnung der Folie 800 bei der Aktivierung (Übergang in die zweite Stellung) beschränkt wird und andererseits die einzuleitende, allein zur Dehnung der Folie 800 aufgewandte Aktivierungskraft minimiert wird und dabei ein ringförmiger Fluidraum nach Aktivierung zwischen Folie 800 und Gewölbe der Kappe 801 verbleibt, der die Durchbrüche 805 bei ausreichend geringem Strömungswiderstand untereinander und mit den zu- und/oder abgehenden Kanälen verbindet.

Eine die Kappe 801 nach unten abschließende Fläche oder Ebene, hier als untere Kappen-Ringstirn 812 bezeichnet, bildet zusammen mit einer vorzugsweise umlaufenden Auflagefläche des Ventilsitzes 803 für die untere Kappen-Ringstirn 812, hier als Ventilsitz-Anschlag-Ringauflage 813 bezeichnet, das funktionale System zur Gewährleistung eines kurzen Aktivierungswegs und präziser konstanter Lage der Kappe 801, wenn sich das Ventil in der zweiten Stellung befindet.

Sowohl auf der oberen Ringstirn 811 als auch im Bereich der Konus-Aufnahme 815 (der einen Zentrierkonus 820 des Ventilkörpers im Gebrauchszustand aufnimmt) weist die Kappe 801 optional zahlreiche Strukturierungen wie die Strukturierungsrillen 816, Nuten und Rücksprünge auf, die benötigt werden, damit die Sterilisationsgase ausreichend viele Flächenanteile der Kappe 801, des Ventilkörpers 802 und der Zylinderwände 807 und 817 des Ventilsitzes 803 erreichen können.

Damit das Bauteil eine symmetrische und verzugsunanfällige Form behält, sind diese äußeren und inneren Strukturierungen in einer konzentrischen und gleichzahligen oder geradzahlig geteilten Anzahl in Relation zur Anzahl der Durchbrüche 805/Rastzungen 806 angeordnet. Da letztere Anzahl bevorzugt ungeradzahlig ist, sieht man in jeder Halbschnitt-Abbildung (oder in jedem Frontalschnitt durch den Mittelpunkt der Kappe 801) auf einer Seite (links oder rechts) eine Rastzunge, ein Gewölbe oder eine Auflagezunge 818 oder einen Schnitt hiervon, während dies auf der gegenüberliegenden Seite aufgrund der Asymmetrie nicht der Fall ist. Diese Darstellung im Schnitt darf allerdings nicht zu der Fehlannahme verleiten, dass an beliebiger Stelle Strukturen oder Auflagen fehlen oder Bauteile nicht abgestützt in der Luft hängen. Vielmehr ergibt sich stets eine gleichmäßige Verteilung von Materialberührung und -nichtberührung über Fläche und Umfang.

Zur Steifheit der Kappe 801 tragen nicht nur die Gewölbestruktur 810, sondern auch vorzugsweise radial außen vorgesehene vertikale Zylinderwände 819 und die vorzugsweise steilkonischen Umlaufwände innerhalb und außerhalb des umlaufenden Spalts, hier als Kappen-Zentrier-Rille 817a bezeichnet, bei. Man erhält ein Gebilde mit relativ gleichmäßiger Wandstärke bei hoher Axialsteifigkeit. Eine axiale Kraft von 60 N auf die obere Ringstirn 811 der Kappe 801 bei Abstützung über die untere Ringstirn 812 der Kappe 801 führt lediglich zu einer Absenkung der vorzugsweise zentralen Konus-Aufnahme 815 von ca. 0,04 mm.

Der Durchmesser der Zylinderwand 807 des Ventilsitzes 803, an welcher die Kante 809 der Kappe 801 in der zweiten, nicht aber in der ersten Stellung anliegt, ist definiert größer ausgelegt als der starre Außendurchmesser der Kappe 801 an der Kante 827 des Gewölbes 810 und größer als der starre Außendurchmesser der Kappe 801 an der verrundeten Umlaufkante zur unteren Ringstirn 812. Mit dieser Spiel-Auslegung kann die Kappe 801 um bis zu 6 Grad verkippt werden, ohne sich an den beiden harten Durchmessern gegen die harte Zylinderwand 807 zu verkanten. Mehr als ca. 3,4 Grad Verkippung der Kappe 801 sind aber mechanisch durch eine verkippte Aktivierung ohnehin nicht realisierbar.

Der Ventilsitz 803 weist eine Raststufe 828 auf, welche sich im Übergang der Zylinderwand 836 zur Kappenaufnahme-Ventilsitz-Zylinderwand 817 befindet. Sie stellt eine Durchmesserverjüngung zum Inneren des Ventilsitzes 803 dar.

Der Ventilkörper 802 hat optional eine rollbalgähnliche Form und umfasst wiederum jeweils optional die Funktionselemente Zentrierkonus 820, welcher vorzugsweise mittig angeordnet ist und zur Kappe 801 hin zu einer Stirnfläche 829 ansteigt oder mit dieser abschließt, Anschlagstirn 821, gegen welche in der zweiten Stellung ein Zentrieranschlagdorn 824a des Ventilsitzes 803 anschlägt, wenigstens einen Hilfszentrierhöcker 822, welcher in wenigstens der zweiten Stellung den Zentrieranschlagdorn 824a berührt, und eine Federmembran 823, welche in Form eines umlaufend geschlossenen (also beispielsweise kreisförmigen) aber nach oben (bezogen auf die Darstellung der Figuren im Einbauzustand) offenen Kanals zwischen dem Dichtring 824 und der Anschlagstirn 821 vorgesehen ist. Der Ventilkörper 802 weist ferner einen nach radial außen ragenden, vorzugsweise umlaufenden Haltering 825 auf, welcher allein überragt werden kann durch die Spitze des Zentrierdorns, und welcher der radial am weitesten vorstehende Abschnitt des Ventilskörpers 803 ist.

In einer bevorzugten Ausführungsform hat die Einbauöffnung des Ventilsitzes 803 einen größten Durchmesser von 13 mm (an der Stelle der Folienauflage).

Der Zentrierkonus 820 ist, wie in Fig. 6c erkennbar ist, in bestimmten beispielhaften Ausführungsformen gemäß der vorliegenden Erfindung ein zentral angeordneter Kegelstumpf. Seine Kegelmantelfläche dient vorzugsweise sowohl als Einführschräge in den zugeordneten Zentrierkonusbereich oder die Konus-Aufnahme 815 beim Zusammenbau, als auch als radiale Zentrierung zur Kappe 801 mit definiertem geringem Restspiel.

Die Anschlagstirn 821 ist in gewissen beispielhaften Ausführungsformen gemäß der vorliegenden Erfindung zentral angeordnet und kann die Bodenfläche des Kegelstumpfs des Zentrierkonus 820 bilden. In Verbindung mit dem Zentrieranschlagdorn oder -dom 824a des Ventilsitzes 803 ergibt sich sowohl eine axiale, gering spielbehaftete Lageeinschränkung des Rückschlagventils als auch ein Bewegungsanschlag bei extremen Betriebsdrücken in Sperr-Richtung des Rückschlagventils.

Die optionalen Hilfszentrierhöcker 822 bilden in bestimmten beispielhaften Ausführungsformen gemäß der vorliegenden Erfindung zusammen mit der oftmals abgerundeten, im Wesentlichen zylindrischen Mantelfläche des Zentrieranschlagdorns oder -doms 824a in vorteilhafter Weise eine weitere, in der Regel leicht spielbehaftete, radiale Zentriereinrichtung. Letztere kann beispielsweise mittels des Montagevorgangs dazu beitragen (oder hierfür verantwortlich sein), dass eine Verkippung des Rückschlagventils im Ventilsitz 803 verhindert, gleichzeitig aber während des Betriebs ein möglichst geringer Einfluss auf das Öffnungsverhalten des Ventils genommen wird.

Die federnde Verbindungsmembran oder Federmembran 823 kann vorteilhaft rollbalg-ähnlich sein. Sie kann zur lagerichtigen Halterung des Dichtrings 824 in beiden Betriebszuständen oder -stellungen von Vorteil sein: Da sie biegsam ist, können sich axiale Vorspannungen und axiale Betriebs-Vorspannungen des Dichtrings 824 gegen die Dichtringzone 826 erzeugen lassen. Dabei kann die Federmembran 823 radial bis hin zum Dichtring 824 eine fluiddichte Zone bilden. Schließlich kann die Federmembran 823 auch den Haltering 825 unter Vorspannung auf den Haltenasen oder Auflagezungen 818 der Kappe 801 halten, und sie kann den Haltering 825 radial während der Montage und während der Betriebsstellung in geöffneter Durchflussrichtung zentrieren.

Wie in den Fig. 6a bis 6d zu erkennen ist, bringt die Kappe 801 den Ventilkörper 802 bereits beim werksseitigen Einsetzen des Ventilkörpers 802 in die Kappe 801 in eine elastisch vorgespannte Stellung, und zwar sobald der Ventilkörper 802 in der Kappe 801 eingerastet ist.

Die dabei erzielte Vorspannkraft ist vorzugsweise knapp unter dem Druck bzw. der Kraft dimensioniert, bei der das Ventil im bestimmungsgemäßen Gebrauch öffnen soll.

Aus Fig. 6c ist für den Fachmann bereits abzusehen was die Fig. 6e bis 6h im Detail zeigen, nämlich dass die Federmembran 823 als Feder oder Rückstellelement wirken kann, wenn der Ventilkörper 802 aus Fig. 6c in die Kappe 801 aus Fig. 6b eingerastet wird. Ist der Ventilkörper 802 in der Kappe 801 eingerastet, stützt sich der mittels der Federmembran 823 vorgespannte Ventilkörper 802 einerseits mit dem Haltering 825 hinter den Auflagezungen 818 ab und andererseits mit dem Zentrierkonus 820 in der Konus-Aufnahme 815. In diesem Zustand ist die Federmembran 823 elastisch vorgespannt, weil der Ventilkörper 802 aufgrund der gewählten Abmessungen von Ventilkörper 802 und Kappe 801 im eingerasteten Zustand durch Formschluss in den elastisch vorgespannten Zustand gezwungen wird.

Wenn die Kappe 801 zusammen mit dem in ihr eingerasteten Ventilkörper 802 in den Ventilsitz 803 aus Fig. 6d eingesetzt wird, so dass die Rastzungen 806 in der Ventilsitzwand oder der Kappenaufnahme-Ventilsitz-Zylinderwand 817 sitzen, dann besteht zwischen der Dichtfläche oder Dichtringzone 826 des Ventilsitzes 803 und dem Dichtring 824 des Ventilkörpers 802 ein Spalt der Breite b. Zwischen den Auflagezungen 818 und der Ventilsitz-Anschlag-Ringauflage 813 besteht dabei ein weiterer Spalt der Breite a. Bestehen beide Spalte (d. h. sind beide offen oder nachweisbar), so liegt die erste, zum Gassterilisieren geeignete Stellung dieser erfindungsgemäßen, beispielhaften Ausführungsform vor. Der Spalt b kann kleiner oder schmaler als der Spalt a sein.

Durch Aufprägen eines Weges/einer Verschiebung und/oder einer Kraft größer als die Vorspannkraft auf die Kappe 801, bewirkt durch Drücken eines Aktors 951 auf die Folie 800, kann der Spalt b geschlossen werden, wenn oder indem der Dichtring 824 dichtend an die Dichtfläche 826 gedrückt wird. Dabei wird davon ausgegangen, dass die Kassette 900 mit dem Ventilsitz 803 fest gelagert ist und die Kappe 801 mit dem Ventilkörper 802 relativ zum Ventilsitz 803 bewegbar/verschiebbar ist. Ist der Spalt b geschlossen, liegt die zweite Stellung der beispielhaften, vorliegenden Ausführungsform vor.

Die Kappe 801 ist mit dem in sie eingesetzten Ventilkörper 802 aufgrund der Durchbrüche 805 der Kappe 801 flüssigkeitsdurchlässig, so dass Flüssigkeit von der Seite und/oder von oben durch die Kappe 801 dringen kann.

Die erforderliche Wegaufprägung/Verschiebung zum Öffnen und Schließen des Ventils ist sehr klein, z. B. nur 0,8 mm zwischen geschlossener und geöffneter Stellung bei dem Ausführungsbeispiel gemäß den Fig. 6a bis 6d. Dies ermöglicht eine sehr flache Federkennlinie der Federmembran 823 in Verbindung mit der Vorspannung. Die gewünschte Dichtkraft mit welcher der Dichtring 824 dichtend an die Dichtfläche 826 gedrückt werden soll, wird so bereits mit sehr kleinen Wegaufprägungen erzielt und ändert sich mit zunehmender Wegaufprägung nur wenig. Sobald aufgrund einer Wegaufprägung der Dichtring 824 an die Dichtfläche 826 stößt, steht zwischen Dichtring 824 und Dichtfläche 826 entsprechend einer Sprungfunktion sprungartig die Dichtkraft gemäß Vorspannung zur Verfügung.

Die **Fig. 6e** bis **6h** zeigen die Ausführungsform der Fig. 6a bis 6d in zusammengefügtem Zustand in verschiedenen Ventilstellungen. Fig. 6e zeigt sie in der ersten, initialen Stellung vor dem Schließen einer Maschinentür, Fig. 6f in der zweiten, aktivierten Stellung bei geschlossener Maschinentür bei maximalem Volumenstrom des Behandlungsfluids, Fig. 6g in der zweiten, aktivierten Stellung bei geschlossener Maschinentür bei maximalem Sperrdruck des Behandlungsfluids, und Fig. 6h in der aktivierten, zweiten Stellung bei geöffneter Maschinentür, nachdem die Kassette 900 abgerüstet, d. h. von der Behandlungsvorrichtung entfernt wurde.

In den Fig. 6e bis 6h weisen die großen Pfeile auf Volumenströme hin, die kleinen auf Spalte, die sich zwischen den betroffenen Komponenten ergeben können.

Die Vorspannung des Ventilkörpers 802 ergibt sich im Beispiel der Fig. 6a bis 6h durch das Abstützen des Ventilkörpers 802 mittels seines Haltrings 825 auf den Auflagezungen 818 der Kappe 801. Zudem liegt sein Zentrierkonus 820 an der Konus-Aufnahme 815 an. In der in Fig. 6h gezeigten Stellung stützt sich der Ventilkörper 802 hingegen nicht mehr auf den Auflagezungen 818 ab. Eine Verspannung des Ventilkörpers 802 ergibt sich nun unter Beteiligung des Dichtrings 824 des Ventilskörpers 802, welcher sich an der Dichtringzone 826 des Ventilsitzes 803 abstützt.

### Bezugszeichenliste

- 1: Rückschlagventilanordnung
- 21: Durchmesserabsatz
- 30: Ventilkörper
- 31: Kernzylinder
- 32: Radialdichtsteg
- 32a: umlaufende Dichtkante
- 32b: Kelchmantel
- 32c: umlaufender Spalt
- 32d: Spaltgrund
- 33: Führungs-Klemm-Drainage-Rippen
- 34: Klemm-Führungs-Noppen
- 35: Klemm-Rast-Noppen
- 36: Drainage-Fußrippen
- 37: Dichtzylinder
- 38: Führungszylinder
- 39: Raststufe
- 40: Ventilkörper
- 41: Positionierungsring
- 41a: Drainagestrukturen
- 41b: obere Ringstirnfläche
- 41c: untere Ringstirnfläche
- 42: Durchlassringzone
- 43: Dichtringzone
- 44: Kernbereich
- 45: konischer Dichtsitz
- 46: Stössel
- 50: Ventilkörper
- 50a: Drainagestrukturen
- 51: Kappe
- 51a: Drainagestrukturen
- 51b: Aktivierungsstirnfläche
- 52: Dichtring
- 53: Noppen
- 54: Durchbruch
- 55: Rippen des Ventilfußes
- 57: Haltekopf
- 58: Ringstirnfläche
- 59: Ringstirnfläche
- 60: Ventilkörper
- 60a: Ventilboden
- 60b: Ringstirn des Ventilkörpers
- 61: Kappe
- 61a: Ringfläche
- 61b: folienzugewandte Ringstirnfläche
- 61c: folienabgewandte Ringfläche
- 62: Zuganker
- 63: Zungen
- 70: Kappe, examplarisch ausgestaltet als Rastkappe
- 70a: untere Ringstirn
- 70b: Ventilkörper
- 71: Haltenasen
- 72: Heißkanal-Anspritzungen
- 73: konischer Dorn
- 74: Verbindungsmembran
- 75-76-77: Halte-Dicht-Ring
- 76: Auflageflansch des Halte-Dicht-Rings 75-76-77
- 78: Membran
- 800: Folie
- 801: Kappe
- 802: Ventilkörper
- 803: Ventilsitz
- 803a: Ringfläche
- 804: Rohranordnung
- 805: Durchbrüche
- 806: Rastzungen
- 807: Kappenaufnahme-Ventilsitz-Zylinderwand
- 808: Fluidkanal
- 809: scharfe Kante
- 810: statische Stützbögen oder Gewölbestruktur
- 811: obere Kappen-Ringstirn
- 812: untere Kappen-Ringstirn
- 813: Ventilsitz-Anschlag-Ringauflage
- 814: Foliensteg
- 815: Konus-Aufnahme
- 816: Strukturierungsrillen
- 817: Kappenaufnahme-Ventilsitz-Zylinderwand
- 817a: Kappen-Zentrier-Rille
- 818: Auflagezunge
- 819: vertikale Zylinderwände
- 820: Zentrierkonus
- 821: Anschlagstirn
- 822: Hilfszentrierhöcker
- 823: Federelement, ausgestaltet als Federmembran
- 824: Dichtring
- 824a: Zentrieranschlagdorn oder -dom
- 825: Haltering
- 826: Dichtringzone
- 827: scharfe Kante
- 828: Raststufe des Ventilsitzes
- 829: Stirnfläche
- 830: Hartkörper
- 831: konnektorseitiger Raum
- 836: Zylinderwand
- 837: Ventilsitz-Stützstirn
- 900: Kassette oder medizinische Funktionsvorrichtung
- 950: Aktor-Sensor-Matte
- 951: Aktor
- 1000: Dialysemaschine, Maschine, Blutbehandlungsvorrichtung

## Patentansprüche

1. Medizinische Funktionsvorrichtung (900) mit einem Rückschlagventil (1) und einem Ventilsitz (803) für das Rückschlagventil (1), wobei das Rückschlagventil (1) ausgestaltet ist, um neben einer ersten, zum Gassterilisieren geeigneten Stellung, bei welcher ein Sterilisierungsspalt verbleibt, mittels Aufbringens einer Kraft auf einen Abschnitt des Rückschlagventils (1) und/oder mittels dessen Verschiebung eine zweite Stellung einzunehmen, in welcher das Rückschlagventil (1) eine Rückschlagfunktion ausübt,
**dadurch gekennzeichnet, dass**
das Rückschlagventil (1) ausgestaltet ist, um nach Wegfall der Kraft und/oder der verschiebenden Wirkung nach erfolgtem Überführen in die zweite Stellung in der zweiten Stellung zu verbleiben,
wobei entweder
- das Rückschlagventil (1) einen Ventilkörper (30; 40; 50; 60; 70b; 802) und eine hiervon getrennt gefertigte Kappe (51; 61; 70; 801) aufweist, wobei sich der Ventilkörper (30; 40; 50; 60; 70b; 802) durch Aufbringen der Kraft oder mittels des verschiebenden Aktors elastisch verformt in der Kappe (51; 61; 70; 801) verrastet, verklemmt oder verkeilt;
oder
- ein Positionierungsring (41) des Ventilkörpers (30; 40; 50; 60; 70b; 802) Drainagestrukturen (41a; 50a; 51a) aufweist; wobei eine Mantelfläche des Positionierungsrings (41) einen größeren Durchmesser aufweist als eine zugeordnete Absatzbohrung in der medizinischen Funktionsvorrichtung (900);
oder
- innerhalb eines zylinderförmigen Abschnitts des Ventilsitzes (803) der Kassette (900) eine Raststufe (828) oder eine stufenartige Durchmesserverjüngung ausgestaltet ist.

2. Funktionsvorrichtung (900) nach Anspruch 1, wobei das Rückschlagventil (1) ausgestaltet ist, um mittels eines Aktors (951) einer Blutbehandlungsvorrichtung (1000), zu deren Betrieb die Funktionsvorrichtung (900) mit der Blutbehandlungsvorrichtung (1000) verbunden wird, von der ersten Stellung in die zweite Stellung übergeführt zu werden, wobei das Rückschlagventil (1) nach Wegfall der Kraft oder nach Rückzug des überführenden oder verschiebenden Aktors (951) aufgrund von Reibschluss in der zweiten Stellung verbleibt.

3. Funktionsvorrichtung (900) nach einem der Ansprüche 1 bis 2, wobei der Ventilkörper (30; 40; 50; 60; 70b; 802) eine Anzahl von Noppen aufweist, welche nach Verbinden des Ventilkörpers (30; 40; 50; 60; 70b; 802) mit der Kappe (51; 61; 70; 801) radial aus Durchbrüchen (54; 805) der Kappe (51; 61; 70; 801) nach außen ragen.

4. Funktionsvorrichtung (900) nach Anspruch 1 bis 3, wobei der Ventilsitz (803), gegen welchen der Ventilkörper (30; 40; 50; 60; 70b; 802) und/oder die Kappe (51; 61; 70; 801) in der zweiten Stellung dichten, im Dichtungsbereich konisch, zylindrisch oder flach und vorzugsweise umlaufend ausgestaltet ist.

5. Funktionsvorrichtung (900) nach einem der Ansprüche 1 bis 4, wobei der Ventilkörper (30; 40; 50; 60; 70b; 802) radial unter einer ersten Vorspannung und axial unter einer zweiten Vorspannung in der Kappe (51; 61; 70; 801) sitzt, wobei die zweite Vorspannung größer als die erste ist.

6. Funktionsvorrichtung (900) nach einem der Ansprüche 1 bis 4, wobei der Ventilkörper (30; 40; 50; 60; 70b; 802) vorzugsweise die Form eines Behälters oder Napfes hat und mit einem Ventilboden (60a) ausgestaltet ist, welcher einen, optional zentral und steif an den Ventilboden (60a) angebundenen, Zuganker (62) aufweist, wobei der Zuganker (62) ausgestaltet ist zum Verbinden des Ventilkörpers (30; 40; 50; 60; 70b; 802) mit der Kappe (51; 61) indem der Zuganker (62) in eine Rastöffnung der Kappe (51; 61; 70; 801) eingerastet wird.

7. Funktionsvorrichtung (900) nach einem der Ansprüche 1 bis 6, wobei die Kappe (51; 61; 70; 801) die Form eines Gewölbes mit mehreren radial außen und axial oben offenen Durchbrüchen (54; 805) aufweist, wobei in den Durchbrüchen (54; 805) Rastzungen (806) angeordnet sind, die, vorzugsweise auch wenn sie radial nach innen gebogen sind oder werden, die Durchbrüche (54; 805) nur teilweise verschließen.

8. Funktionsvorrichtung (900) nach einem der vorangegangenen Ansprüche 1 bis 7, wobei die Kappe (51; 61; 70; 801) eine umlaufende Kante (809) aufweist, die sich auf Höhe der Rastzungen (806) befindet und dabei die Haupttrennebene des Spritzgusswerkzeugs bildet.

9. Funktionsvorrichtung (900) nach einem der vorangegangenen Ansprüche, wobei in der ersten Stellung ein umlaufender Sterilisations-Spalt verbleibt, welcher in der zweiten Stellung geschlossen ist, und wobei die axiale Einbaulage und damit der Sterilisations-Spalt durch Reibschluss zwischen Komponenten, beispielsweise an Klemm-ZentrierRippen, aufrecht gehalten wird.

10. Funktionsvorrichtung (900) nach einem der vorangegangenen Ansprüche, ausgestaltet als Blutkassette, Kassette, Blutschlauch, Infusionsschlauch.

11. Funktionsvorrichtung (900) nach Anspruch 1 bis 15, vorzugsweise ausgestaltet als Blutkassette (900), welche einen Hartkörper (830) und eine den Hartkörper (830) oder Teile hiervon abdeckende Folie (800) aufweist, wobei der Ventilsitz (803) im Hartkörper (830) vorgesehen ist, und wobei das Rückschlagventil (1) angeordnet ist, um mittels Druck auf die Folie (800) und/oder Verschiebung des Aktors (951) der Blutbehandlungsvorrichtung (1000) auf die Folie (800) hin in die zweite Stellung übergeführt zu werden.

12. Funktionsvorrichtung (900) nach Anspruch 11, wobei die folienseitige Stirnfläche (61b) des Rückschlagventils (1) nicht über die Folienebene der Blutkassette (900) hinausragt.

13. Funktionsvorrichtung (900) nach einem der vorangegangenen Ansprüche, wobei das Rückschlagventil (1) oder ein Abschnitt hiervon ausgestaltet ist, um eine Vorspannung des in den Ventilsitz (803) eingefügten Rückschlagventils (1) oder wenigstens eines Abschnitts hiervon zu bewirken.

14. Funktionsvorrichtung (900) nach Anspruch 13, wobei die Vorspannung mittels eines Federelements (823) bewirkt wird, welche beispielsweise als Federmembran (823) ausgestaltet ist.

15. Funktionsvorrichtung (900) nach Anspruch 14, wobei das Federelement (823) ein Abschnitt des Ventilkörpers (802) ist.

## Claims

1. A medical functional device (900) comprising a check valve (1) and a valve seat (803) for the check valve (1), wherein the check valve (1) is designed to assume, in addition to a first position suitable for gas sterilization, in which a sterilization gap remains, a second position in which the check valve (1) performs a non-return function by applying a force to a section of the check valve (1) and/or by shifting it,
**characterized in that**
the check valve (1) is designed to remain in the second position after release of the force and/or after the shifting effect following an accomplished transfer into the second position,
wherein either
- the check valve (1) comprises a valve body (30; 40; 50; 60; 70b; 802) and a cap (51; 61; 70; 801) manufactured separately therefrom, wherein the valve body (30; 40; 50; 60; 70b; 802) latches, jams or wedges with elastic deformation into the cap (51; 61; 70; 801) by applying the force or by means of the shifting actuator;
or
- a positioning ring (41) of the valve body (30; 40; 50; 60; 70b; 802) comprises drainage structures (41a; 50a; 51a); wherein a sheath surface of the positioning ring (41) comprises a larger diameter than an associated stepped hole in the medical functional device (900);
or
- a snap-in stage (828) or a step-like diameter taper is designed within a cylindrical section of the valve seat (803) of the cassette (900).

2. The functional device (900) according to claim 1, wherein the check valve (1) is designed to be transferred from the first position into the second position by means of an actuator (951) of a blood treatment apparatus (1000), for the operation of which the functional device (900) is connected to the blood treatment apparatus (1000), wherein the check valve (1) remains in the second position due to friction closure after release of the force or after retraction of the transferring or shifting actuator (951).

3. The functional device (900) according to anyone of the claims 1 to 2, wherein the valve body (30; 40; 50; 60; 70b; 802) comprises a number of knobs which radially protrude outwards from apertures (54; 805) of the cap (51; 61; 70; 801) after connecting the valve body (30; 40; 50; 60; 70b; 802) with the cap (51; 61; 70; 801).

4. The functional device (900) according to claim 1 to 3, wherein the valve seat (803) against which the valve body (30; 40; 50; 60; 70b; 802) and/or the cap (51; 61; 70; 801) seals in the second position, is designed conically, cylindrically or flatly and preferably circumferentially in the sealing area.

5. The functional device (900) according to anyone of the claims 1 to 4, wherein the valve body (30; 40; 50; 60; 70b; 802) is seated radially under a first pre-tension and axially under a second pre-tension in the cap (51; 61; 70; 801), wherein the second pre-tension is larger than the first one.

6. The functional device (900) according to anyone of the claims 1 to 4, wherein the valve body (30; 40; 50; 60; 70b; 802) preferably has the form of a container or a cup and is equipped with a valve tray (60a) comprising a tension rod (62) which is optionally fixed centrally and rigidly to the valve tray (60a), wherein the tension rod (62) is designed for connecting the valve body (30; 40; 50; 60; 70b; 802) with the cap (51; 61) by engaging the tension rod (62) in a snap-in opening of the cap (51; 61; 70; 801).

7. The functional device (900) according to anyone of the claims 1 to 6, wherein the cap (51; 61; 70; 801) has the form of an arch having several apertures (54; 805) opening radially outwards and axially upwards, wherein snap-in tongues (806) are arranged in the apertures (54; 805), preferably even if they are or get bent radially inwards and partially close the apertures (54; 805).

8. The functional device (900) according to anyone of the preceding claims 1 to 7, wherein the cap (51; 61; 70; 801) presents a peripheral edge (809) located at the level of the snap-in tongues (806) and thereby forms the main parting plane of the injection molding tool.

9. The functional device (900) according to anyone of the preceding claims, wherein a circumferential sterilization gap remains in the first position, which is closed in the second position, and wherein the axial mounting position and thus the sterilization gap is maintained by friction closure between components, e.g. on clamping centering ribs.

10. The functional device (900) according to anyone of the preceding claims, designed as blood cassette, cassette, blood tube, infusion tube.

11. The functional device (900) according to claim 1 to 10, preferably designed as a blood cassette (900), which comprises a hard body (830) and a film (800) covering the hard body (830) or parts thereof, wherein the valve seat (803) is provided in the hard body (830), and wherein the check valve (1) is arranged to be transferred into the second position by means of pressure on the film (800) and/or shifting the actuator (951) of the blood treatment apparatus (1000) towards the film (800).

12. The functional device (900) according to claim 11, wherein the film-sided front face (61b) of the check valve (1) does not project beyond the film plane of the blood cassette (900) .

13. The functional device (900) according to anyone of the preceding claims, wherein the check valve (1) or a section thereof is designed to effect a pre-tension of the check valve (1) inserted into the valve seat (803) or of at least a section thereof.

14. The functional device (900) according to claim 13, wherein the pre-tension is effected by means of a spring element (823), which is designed, for example, as a spring membrane.

15. The functional device (900) according to claim 14, wherein the spring element (823) is a section of the valve body (802) .

## Revendications

1. Un dispositif fonctionnel médical (900) comprenant un clapet anti-retour (1) ainsi qu'un siège de clapet (803) pour le clapet anti-retour (1), où le clapet anti-retour (1) est conçu pour adopter, en plus d'une première position adaptée à une stérilisation au gaz, dans laquelle une fente de stérilisation subsiste, une seconde position dans laquelle le clapet anti-retour (1) exerce une fonction anti-retour, par application d'une force sur une partie du clapet anti-retour (1) et/ou par déplacement de celui-ci,
**caractérisé en ce que**
le clapet anti-retour (1) est conçu pour rester dans la seconde position après relâchement de la force et/ou de l'effet de déplacement à la suite d'un transfert effectué dans la seconde position,
où soit
- le clapet anti-retour (1) comprend un corps de clapet (30; 40; 50; 60; 70b; 802) ainsi qu'un capuchon (51; 61; 70; 801) fabriqué séparément, où le corps de clapet (30; 40; 50; 60; 70b; 802) s'encliquète, se coince ou se cale en se déformant élastiquement dans le capuchon (51; 61; 70; 801) par application de la force ou au moyen de l'actionneur mobile;
soit
- un anneau de positionnement (41) du corps de clapet (30; 40; 50; 60; 70b; 802) comprend des structures de drainage (41a; 50a; 51a), où une surface de gaine de l'anneau de positionnement (41) présente un diamètre plus grand qu'un orifice étagé associé dans le dispositif fonctionnel médical (900);
soit
- une marche d'encliquetage (828) ou une réduction par paliers du diamètre est conçue à l'intérieur d'une partie cylindrique du siège de clapet (803) de la cassette (900).

2. Le dispositif fonctionnel (900) selon la première revendication, où le clapet anti-retour (1) est conçu pour être transféré de la première position à la seconde position au moyen d'un actionneur (951) d'un appareil de traitement du sang (1000), pour le fonctionnement duquel le dispositif fonctionnel (900) est relié à l'appareil de traitement du sang (1000), où le clapet anti-retour (1) reste dans la seconde position après relâchement de la force ou après rétraction de l'actionneur (951) de transfert ou de déplacement, en raison du contact de friction.

3. Le dispositif fonctionnel (900) selon l'une quelconque des revendications 1 à 2, où le corps de clapet (30; 40; 50; 60; 70b; 802) comprend un certain nombre de protubérances faisant saillie radialement vers l'extérieur depuis des ouvertures (54; 805) du capuchon (51; 61; 70; 801) après raccordement du corps de clapet (30; 40; 50; 60; 70b; 802) avec le capuchon (51; 61; 70; 801).

4. Le dispositif fonctionnel (900) selon une revendication 1 à 3, où le siège de clapet (803), contre lequel le corps de clapet (30; 40; 50; 60; 70b; 802) et/ou le capuchon (51; 61; 70; 801) assure(nt) l'étanchéité dans la seconde position, est conçu de manière conique, cylindrique ou plate et de préférence de manière périphérique dans la zone d'étanchéité.

5. Le dispositif fonctionnel (900) selon l'une quelconque des revendications 1 à 4, où le corps de clapet (30; 40; 50; 60; 70b; 802) est maintenu en place radialement sous une première précontrainte et axialement sous une seconde précontrainte dans le capuchon (51; 61; 70; 801), la seconde précontrainte étant plus grande que la première.

6. Le dispositif fonctionnel (900) selon l'une quelconque des revendications 1 à 4, où le corps de clapet (30; 40; 50; 60; 70b; 802) a de préférence la forme d'un récipient ou d'une coupelle et est muni d'un fond de clapet (60a) qui présente un tirant d'ancrage (62) fixé optionnellement de manière centrale et rigide au fond de clapet (60a), le tirant d'ancrage (62) étant conçu pour relier le corps de clapet (30; 40; 50; 60; 70b; 802) au capuchon (51 ; 61) par encliquetage du tirant d'ancrage (62) dans une ouverture d'encliquetage du capuchon (51 ; 61 ; 70 ; 801).

7. Le dispositif fonctionnel (900) selon l'une quelconque des revendications 1 à 6, où le capuchon (51; 61; 70; 801) a la forme d'une voûte ayant plusieurs passages (54; 805) ouverts radialement vers l'extérieur et axialement vers le haut, où des languettes d'encliquetage (806) agencées dans les passages (54; 805) ne ferment de préférence que partiellement les passages (54 ; 805), même si elles sont courbées ou se courbent radialement vers l'intérieur.

8. Le dispositif fonctionnel (900) selon l'une quelconque des revendications 1 à 7 qui précédent, où le capuchon (51; 61; 70; 801) présente un bord périphérique (809) situé au niveau des languettes d'encliquetage (806) qui forme ainsi le plan de séparation principal de l'outil de moulage par injection.

9. Le dispositif fonctionnel (900) selon l'une quelconque des revendications précédentes, où une fente de stérilisation périphérique subsiste dans la première position, fente qui est fermée dans la seconde position, et où la position de montage axiale, et donc la fente de stérilisation, est maintenue par friction entre les composants, par exemple sur des nervures de centrage de serrage.

10. Le dispositif fonctionnel (900) selon l'une quelconque des revendications précédentes, conçu comme une cassette à sang, une cassette, un tuyau sanguin, une tubulure de perfusion.

11. Le dispositif fonctionnel (900) selon une revendication 1 à 10, conçu de préférence sous la forme d'une cassette à sang (900), qui comporte un corps rigide (830) ainsi qu'un film (800) recouvrant le corps rigide (830) ou des parties de celui-ci, où le siège de clapet (803) est prévu dans le corps rigide (830), et où le clapet anti-retour (1) est agencé pour être transféré dans la seconde position au moyen d'une pression sur le film (800) et/ou d'un déplacement de l'actionneur (951) de l'appareil de traitement du sang (1000) sur le film (800).

12. Le dispositif fonctionnel (900) selon la revendication 11, où la face frontale (61b) côté film du clapet anti-retour (1) ne dépasse pas au-delà du plan du film de la cassette à sang (900).

13. Le dispositif fonctionnel (900) selon l'une quelconque des revendications précédentes, où le clapet anti-retour (1) ou une partie de celui-ci est conçu(e) pour effectuer une précontrainte du clapet anti-retour (1) inséré dans le siège de clapet (803) ou d'au moins une partie de celui-ci.

14. Le dispositif fonctionnel (900) selon la revendication 13, où la précontrainte est effectuée au moyen d'un élément ressort (823), conçu, par exemple, comme une membrane à ressort.

15. Le dispositif fonctionnel (900) selon la revendication 14, où l'élément ressort (823) est une partie du corps de clapet (802).
